# EUROPÄISCHE PATENTSCHRIFT

(11) **EP 1 385 820 B1**
(45) Veröffentlichungstag und Bekanntmachung des Hinweises auf die Patenterteilung: **12.10.2011**
(21) Anmeldenummer: 02742898.6
(22) Anmeldetag: 13.04.2002
(51) Int. Cl.: C07C 311/21, C07C 311/29, C07D 213/56, C07C 311/08, C07D 233/64, C07D 235/16, C07D 307/54, C07D 277/30, C07D 215/14, C07D 231/12, A61K 31/18

(54) **ANTHRANILSÄUREAMIDE, VERFAHREN ZU IHRER HERSTELLUNG, IHRE VERWENDUNG ALS ANTIARRHYTHMIKA SOWIE PHARMAZEUTISCHE ZUBEREITUNGEN DAVON**
ANTHRANILIC ACID AMIDES, METHOD FOR THE PRODUCTION THEREOF, THEIR USE AS ANTIARRHYTHMIA AGENTS, AND PHARMACEUTICAL PREPARATIONS THEREOF
AMIDES D'ACIDE ANTHRANILIQUE, LEUR PROCEDE DE PRODUCTION, LEUR UTILISATION EN TANT QU'ANTIARYTHMISANTS ET PREPARATIONS PHARMACEUTIQUES LES RENFERMANT

(30) Priorität: 28.04.2001 DE 10121003
(43) Veröffentlichungstag der Anmeldung: 04.02.2004
(73) Patentinhaber: Sanofi-Aventis Deutschland GmbH, 65929 Frankfurt am Main (DE)
(72) Erfinder: BRENDEL, Joachim, 61118 Bad Vilbel (DE); Dr. PIRARD, Bernard, 64263 Darmstadt (DE); PEUKERT, Stefan, 60313 Frankfurt (DE); KLEEMANN, Heinz-Werner, 65474 Bischofsheim (DE); HEMMERLE, Horst, Moblesville, IN 46060 (US)
(74) Vertreter: Michalski Hüttermann & Partner Patentanwälte
(86) Internationale Anmeldenummer: PCT/EP2002/004138
(87) Internationale Veröffentlichungsnummer: WO 2002/088073

(56) Entgegenhaltungen:
- DE-A- 19 947 457
- US-A- 3 497 529
- US-B1- 6 221 866
- OTT H ET AL: "TETRAHYDROISOQUINO[2,1-D][1,4]BENZODIAZEP INES, SYNTHESIS AND NEUROPHARMACOLOGICAL ACTIVITY" JOURNAL OF MEDICINAL CHEMISTRY, AMERICAN CHEMICAL SOCIETY. WASHINGTON, US, Bd. 11, Juni 1968 (1968-06), Seiten 777-787, XP002089079 ISSN: 0022-2623
- DATABASE CHEMABS [Online] Chemical Abstracts Service, Columbus Ohio; SUNA, E; TRAPENCIERIS, P: "Synthesis of racemic 1,2,3,4-tetrahydroisoquinolines and their resolution" XP002212428 & CHEM. HETEROCYCL.COMPD. (N.Y.), Bd. 36, Nr. 3, 2000, Seiten 287-300,
- DATABASE CHEMCATS [Online] CHEMICAL ABSTRACTS SERVICE,COLUMBUS, OHIO, US; XP002212429 & "ENAMINE PRODUCT LISTING" 15. November 2001 (2001-11-15) , AMBINTER , 46, QUAI LOUIS BLERIOT, PARIS; F-75016, FRANCE
- DATABASE CHEMCATS [Online] CHEMICAL ABSTRACTS SERVICE,COMLUMBUS,OHIO,US; XP002212430 & "Ambinter: Exploratory Library" 21. Januar 2002 (2002-01-21) , AMBINTER , 46,QUAI LOUIS BLERIOT,PARIS, F-75016,FRANCE
- DATABASE CHEMCATS [Online] CHEMICAL ABSTRACTS SERVICE,COLUMBUS,OHIO,US; XP002212431 & "Pharma Library Collection" 14. Mai 2001 (2001-05-14) , NANOSYN COMBINATORIAL SYNTHESIS INC. , 625 CLYDE AVE, MOUNTAIN VIEW, CA, 94043-2213, USA

## Beschreibung

Die Erfindung betrifft Verbindungen der Formel I, 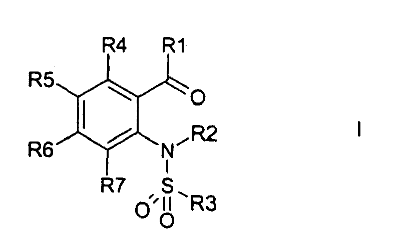 worin R(1), R(2), R(3), R(4), R(5), R(6) und R(7) die im folgenden angegebenen Bedeutungen haben, ihre Herstellung und ihre Verwendung, insbesondere in Arzneimitteln.

Die erfindungsgemäßen Verbindungen der Formel I sind bisher nicht bekannt. Sie wirken auf den sogenannten Kv1.5-Kalium-Kanal und inhibieren einen als "uttra-rapidly activating delayed rectifier" bezeichneten Kaliumstrom im humanen Herzvorhof. Die Verbindungen sind deshalb ganz besonders geeignet als neuartige antiarrhythmische Wirkstoffe, insbesondere zur Behandlung und Prophylaxe von Vorhof-Arrhythmien, z.B. Vorhof-Flimmern (atriale Fibrillation, AF) oder Vorhof-Flattern (atriales Flattern).

Vorhof-Flimmern (AF) und Vorhof-Flattern sind die häufigsten anhaltenden Herzarrhythmien. Das Auftreten erhöht sich mit zunehmenden Alter und führt häufig zu fatalen Folgeerscheinungen, wie zum Beispiel Gehirnschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer Medikamente zur Behandlung atrialer Arrhythmien (S. Nattel, Am. Heart J. 130, 1995, 1094 - 1106; "Newer developments in the management of atrial fibrillation").

Es wurde gezeigt, dass den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindern (T. J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, der aus 3 verschiedenen Komponenten besteht: [Kᵣ, IKₛ und IKᵤᵣ.

Die meisten bekannten Klasse III- Antiarrhythmika (z. B. Dofetilide, E4031 und d-Sotalol) blockieren überwiegend oder ausschließlich den schnell aktivierenden Kaliumkanal IKᵣ, der sich sowohl in Zellen des menschlichen Ventrikel als auch im Vorhof nachweisen lässt. Es hat sich jedoch gezeigt, dass diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B - 49B; "Current status of class III antiarrhythmic drug therapy"). Neben diesem hohen, zum Teil tödlichen Risiko bei niedriger Frequenz, wurde für die IKᵣ-Blocker ein Nachlassen der Wirksamkeit unter den Bedingungen von Tachykardie, in der die Wirkung gerade benötigt wird, festgestellt ("negative use-dependence").

Während einige dieser Nachteile durch Blocker der langsam aktivierenden Komponente (IKₛ) möglicherweise überwunden werden können, wurde deren Wirksamkeit bisher nicht bewiesen, da keine klinischen Untersuchungen mit IK_{S}-Kanalblockern bekannt sind.

Die "besonders schnell" aktivierende und sehr langsam inaktivierende Komponente des delayed Rectifier IKᵤᵣ (=ultra-rapidly activating delayed rectifier), die dem Kv1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von IKᵣ bzw. IKₛ eine besonders effektive Methode zur Verlängerung des atrialen Aktionspotentials und damit zur Beendigung bzw. Verhinderung von atrialen Arrhythmien dar. Mathematische Modelle des menschlichen Aktionspotentials legen nahe, dass der positive Effekt einer Blockade des IKᵤᵣ gerade unter den pathologischen Bedingungen einer chronischen atrialen Fibrillation besonders ausgeprägt sein sollte (M. Courtemanche, R. J. Ramirez, S. Nattel, Cardiovascular Research 1999, 42, 477-489: "Ionic targets for drug therapy and atrial fibrillation-induced electrical remodeling: insights from a mathematical model").

Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣStroms im Gegensatz zur Blockade von IKᵣ oder IKₛ das Risiko einer proarrhythmischen Wirkung auf den Ventrikel von vornherein ausgeschlossen. (Z. Wang et al, Circ. Res. 73, 1993, 1061 - 1076: "Sustained Depolarisation-Induced Outward Current in Human Atrial Myocytes"; G.-R. Li et al, Circ. Res. 78, 1996, 689 - 696: "Evidence for Two Components of Delayed Rectifier K+-Current in Human Ventricular Myocytes"; G. J. Amos et al, J. Physiol. 491, 1996, 31 - 50: "Differences between outward currents of human atrial and subepicardial ventricular myocytes").

Antiarrhythmika, die über eine selektive Blockade des IKᵤᵣ-Stroms bzw. Kv1.5-Kanals wirken, sind auf dem Markt bisher jedoch nicht verfügbar. Für zahlreiche pharmazeutische Wirkstoffe (z.B. Tedisamil, Bupivacaine oder Sertindole) wurde zwar eine blockierende Wirkung auf den Kv1.5-Kanal beschrieben, doch stellt die Kv1.5-Blockade hier jeweils nur eine Nebenwirkung neben anderen Hauptwirkungen der Substanzen dar.

In WO 98 04 521 und WO 99 37 607 werden Aminoindane und Aminotetrahydronaphtaline als Kaliumkanalblocker beansprucht, die den Kv1.5-Kanal blockieren. Ebenfalls als Kv1.5-Blocker werden strukturell verwandte Aminochromane in WO 00 12 077 beansprucht. In der Anmeldung WO 99 62 891 werden Thiazolidinone beansprucht die ebenfalls den Kaliumkanal blockieren. In den Anmeldungen WO 98 18 475 und WO 98 18 476 wird die Verwendung verschiedener Pyridazinone und Phosphinoxide als Antiarrhythmika beansprucht, die über eine Blockade des IKᵤᵣ wirken sollen. Die gleichen Verbindungen wurden ursprünglich jedoch auch als Immunsüppressiva beschrieben (WO 96 25 936). Alle in oben genannten Anmeldungen beschriebenen Verbindungen sind strukturell völlig andersartig als die erfindungsgemäßen Verbindungen dieser Anmeldung. Von allen in den oben genannten Anmeldungen beanspruchten Verbindungen sind uns keine klinischen Daten bekannt. Da erfahrungsgemäß nur ein geringer Teil von Wirkstoffen aus der präklinischen Forschung erfolgreich alle klinischen Hürden bis zum Medikament übersteht, besteht weiterhin ein Bedarf an neuen aussichtsreichen Substanzen.

In der Anmeldung DE19947457 werden 2'-substituierte 1,1'-Biphenyl-2-carbonamide als Kv1.5 Blocker beansprucht. In dem Patent US6221866 werden 3-Sulfamoylbenzamide als Kv1.5 Inhibitoren beschrieben. Die in DE19947457 und US6221866 beschriebenen Verbindungen sind strukturell sehr verschieden von den erfindungsgemäßen Verbindungen der Formel 1.

Es wurde nun überraschenderweise gefunden, dass die erfindungsgemäßen Anthranilsäureamide der Formel I potente Blocker des humanen Kv1.5-Kanals sind. Sie können deshalb verwendet werden als neuartige Antiarrhythmika mit besonders vorteilhaftem Sicherheitsprofil. Insbesondere eignen sich die Verbindungen zur Behandlung supraventrikulärer Arrhythmien, z. B. Vorhof-Flimmern oder Vorhof-Flattern.

Die Verbindungen können eingesetzt werden zur Terminierung von bestehendem Vorhof-Flimmern oder -Flattern zur Wiedererlangung des Sinus-Rhythmus (Kardioversion). Darüber hinaus reduzieren die Substanzen die Anfälligkeit zur Entstehung neuer Flimmer-Ereignisse (Erhalt des Sinus-Rhythmus, Prophylaxe).

Die erfindungsgemäßen Verbindungen sind bisher nicht bekannt. Einige strukturell verwandte Verbindungen sind in den nachfolgend diskutierten Publikationen beschrieben.

So wurden z.B. die Verbindungen A und B in FEBS Letters 421 (1981) 217-220 als Serin-Protease-Hemmer erwähnt. Die Verbindungen C und D sowie ähnliche Derivate sind in J. Med. Chem. 11 (1968) 777-787 beschrieben als Vorstufen für die Synthese von Tetrahydroisochino[2,1-d][1,4]benzodiazepinen.

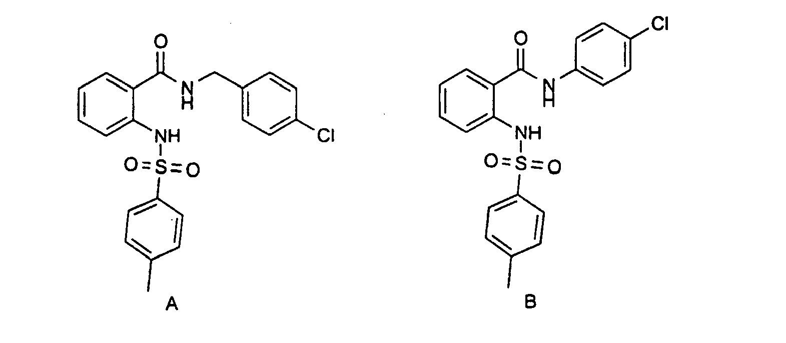

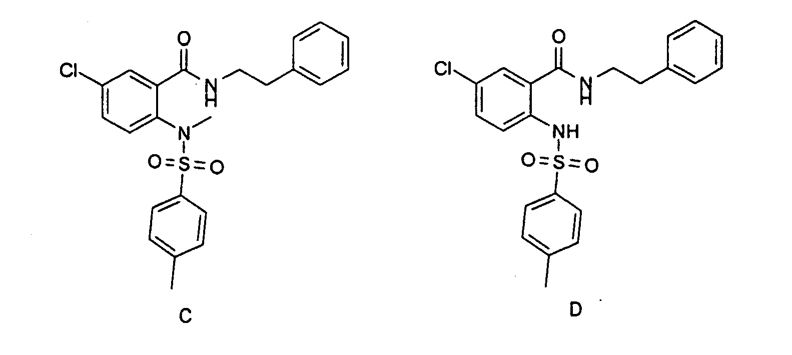

Die Europäische Patentanmeldung EP-OS 0 686 625 beschreibt Anthranilsäurederivate und deren Anwendung als cGMP-Phosphodiesterase-Inhibitoren. Die meisten dort exemplarisch beschriebenen 144 Verbindungen unterscheiden sich von den Verbindungen der Formel I unter anderem durch den Ersatz der Sulfonylgruppe gegen eine Carbonylgruppe. Lediglich 3 der Beispiele enthalten ebenfalls eine Sulfonylaminosubstitution, von denen die Verbindung E (Beispiel 135 in EP-OS 0 686 625) den Verbindungen in dieser Anmeldung strukturell am nächsten kommt. Als mögliche Indikationen für die beanspruchten Verbindungen sind ischämische Herzerkrankungen, Angina pectoris, Hypertension, pulmonare Hypertension, Herzversagen und Asthma genannt. Hierdurch wird in keiner Weise eine Verwendbarkeit von diesen oder ähnlichen Verbindungen als Antiarrhythmika nahegelegt.

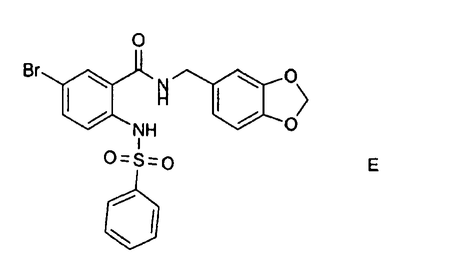

Die Europäische Patentanmeldung EP-OS 0 947 500 beansprucht eine große, teilweise heterogene, Menge von Verbindungen, die als Prostaglandin E2 Antagonisten oder Agonisten wirken sollen. Die meisten der dort enthaltenen Anthranilsäurederivate unterscheiden sich von den Verbindungen dieser Anmeldung unter anderem durch das Vorhandensein einer freien Carbonsäurefunktion.

Die Europäische Patentanmeldung EP-OS 0 491 525 beschreibt Anthranilsäureamide mit verschiedenen 5-Ring-Heterocyclen in der Seitenkette, wie z. B. die Verbindung F, und deren Anwendung zur Behandlung der Diabetes.

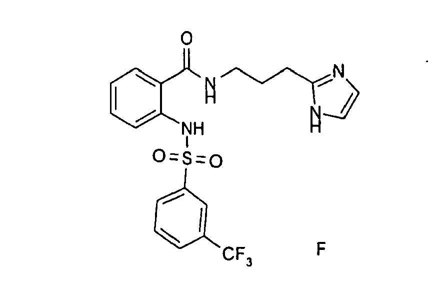

In Ott et al., Journal of Medicinical Chemistry, American Chemical Society, Bd. 11, 1968, 777-778, Suna et al., Chemistry of Heterocyclic Compounds, Vol. 36, 2000, 287-300 und US3497529 werden Anthranilsäureamide als Intermediate zur Herstellung von Tetrahydroisochinobenzodiazepinen, 1,2,3,4-Tetrahydroisochinolinen oder Benzamiden beschrieben. Die in diesen Dokumenten beschriebenen Anthranilsäureamide unterscheiden sich jedoch strukturell von den erfindungsgemäßen Verbindungen der Formel I.

Bei keiner dieser genannten Publikationen ist eine Kaliumkanal blockierende Wirkung genannt, so dass die Wirkung der strukturell verwandten Verbindungen der Formel I nicht erwartet werden konnte.

Die vorliegende Erfindung betrifft Verbindungen der Formel I, 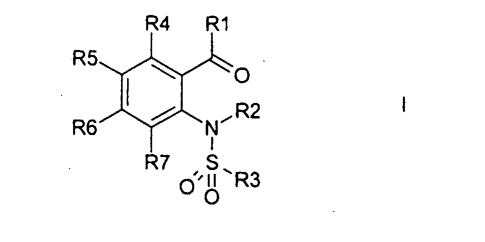 worin bedeuten: 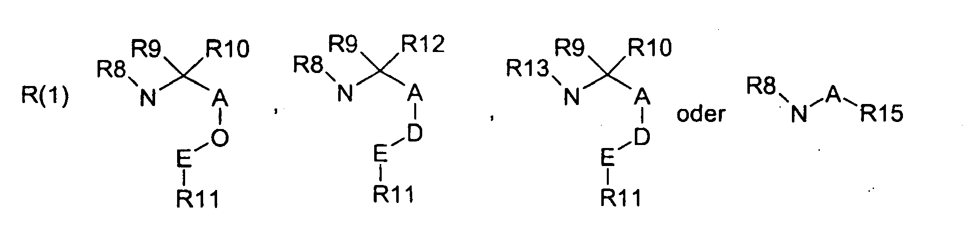
- A: -CnH2n-;
n 0, 1, 2, 3, 4 oder 5;
- O: Sauerstoff;
- D: eine Bindung oder Sauerstoff;
- E: -CₘH₂ₘ-;
m 0, 1, 2, 3, 4 oder 5;
- R(8): Wasserstoff , Alkyl mit 1, 2, 3 oder 4 C-Atomen oder CₚH₂ₚ-R(14);
p 0, 1, 2, 3, 4 oder 5;
R(14) Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(9): Wasserstoff oder Alkyl mit 1, 2, 3, 4, 5 oder 6 C-Atomen;
- R(10): Wasserstoff, Alkyl mit 1, 2, 3 oder 4 C-Atomen, Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(11): Cycloalkyl mit 3, 4, 5 oder 6 C-Atomen, Phenyl, Naphthyl, Thienyl, Furyl, Pyridyl, Pyrazinyl, Pyridazinyl oder Pyrimidyl, wobei Phenyl, Naphthyl, Thienyl, Furyl, Pyridyl, Pyrazinyl, Pyridazinyl und Pyrimidyl unsubstituiert sind oder substituiert mit 1, 2 oder 3 Substituenten ausgewählt aus der Gruppe bestehend aus F, Cl, Br, I, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(12): Alkyl mit 1, 2, 3 oder 4 C-Atomen, Cycloalkyl mit 3, 4, 5 oder 6 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF3, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(13): CpH₂p-R(14');
p 0, 1, 2, 3, 4 oder 5;
R(14') Cycloalkyl mit 3, 4, 5 oder 6 C-Atomen, Tetrahydrofuranyl, Tetrahydropyranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Cycloalkyl mit 3, 4, 5, 6, 7 oder 8 C-Atomen;
- R(2): Wasserstoff oder Alkyl mit 1, 2, 3 oder 4 C-Atomen;
- R(3): Alkyl mit 3, 4, 5, 6 oder 7 C-Atomen, Cycloalkyl mit 3, 4, 5 oder 6 C-Atomen, Phenyl oder Naphthyl, wobei Phenyl oder Naphthyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, Br, I, CF₃, OCF₃, OCHF₂, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Bevorzugt sind Verbindungen der Formel I, worin bedeuten: 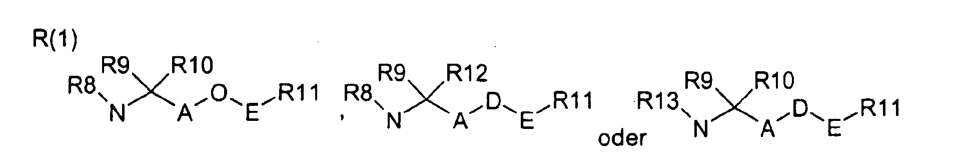
- A: -CₙH₂ₙ-;
n 0, 1, 2, 3, 4 oder 5;
- O: Sauerstoff;
- D: eine Bindung oder Sauerstoff;
- E: -CₘH₂ₘ-;
m 0, 1, 2, 3, 4 oder 5;
- R(8): Wasserstoff , Alkyl mit 1, 2, 3 oder 4 C-Atomen oder CpH₂p-R(14);
p 0, 1, 2, 3, 4 oder 5;
R(14) Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(9): Wasserstoff oder Alkyl mit 1, 2, 3, 4, 5 oder 6 C-Atomen;
- R(10): Wasserstoff, Alkyl mit 1, 2, 3 oder 4 C-Atomen, Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(11): Cycloalkyl mit 3, 4, 5 oder 6 C-Atomen, Phenyl oder Pyridyl, wobei Phenyl und Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(12): Alkyl mit 1, 2, 3 oder 4 C-Atomen, Cycloalkyl mit 3, 4, 5 oder 6 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(13): CpH₂p-R(14');
p 0, 1, 2, 3, 4 oder 5;
R(14') Cycloalkyl mit 3, 4, 5 oder 6 C-Atomen, Tetrahydropyranyl, Tetrahydrofuranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff oder Alkyl mit 1, 2, 3 oder 4 C-Atomen;
- R(3): Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, Br, I, CF₃, OCF₃, OCHF₂, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Besonders bevorzugt sind Verbindungen der Formel I, worin bedeuten: 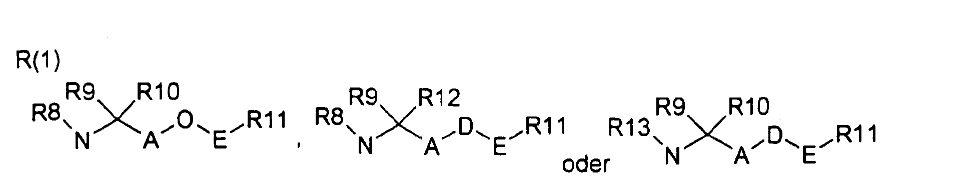
- A: -CnH2n-;
n 0, 1, 2 oder 3;

- O: Sauerstoff;
- D: eine Bindung oder Sauerstoff;
- E: -CₘH₂m-;
m 0, 1, 2 oder 3;
- R(8): Wasserstoff oder Alkyl mit 1, 2, 3 oder 4 C-Atomen;
- R(9): Wasserstoff oder Alkyl mit 1, 2, 3, 4, 5 oder 6 C-Atomen;
- R(10): Wasserstoff, Alkyl mit 1, 2, 3 oder 4 C-Atomen oder Cycloalkyl mit 3, 4 oder 5 C-Atomen,
- R(11): Cycloalkyl mit 3, 4, 5 oder 6 C-Atomen, Phenyl oder Pyridyl, wobei Phenyl und Pyridyl unsubstituiert sind oder substituiert mit 1, 2 oder 3 Substituenten ausgewählt aus der Gruppe bestehend aus F, Cl, Br, CF₃, OCF₃, NO₂, CN, COMe, OH, Alkyl mit 1, 2, 3 oder 4 C-Atomen, Alkoxy mit 1, 2, 3 oder 4 C- Atomen, Dimethylamino, Sulfamoyl, Methylsulfonyl und Methylsulfonylamino;
- R(12): Alkyl mit 1, 2, 3 oder 4 C-Atomen, Cycloalkyl mit 3, 4 oder 5 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(13): CpH₂p-R(14');
p 0, 1, 2, 3, 4 oder 5;
R(14') Cycloalkyl mit 3, 4, 5 oder 6 C-Atomen, Tetrahydrofuranyl, Tetrahydropyranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff oder Alkyl mit 1 oder 2 C-Atomen;
- R(3): Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, Br, CF₃, OCF₃, NO₂, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, Br, CF₃, OCF₃, OCHF₂, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Ganz besonders bevorzugt sind Verbindungen der Formel I, worin bedeuten: 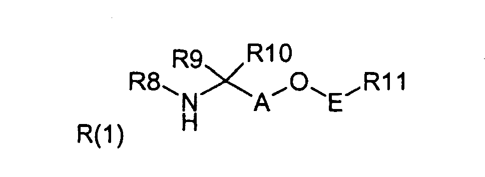
- A: -CₙH₂ₙ-;
n = 0, 1 oder 2;
- O: Sauerstoff;
- E: -CₘH₂ₘ-;
m 0 oder 1;
- R(8): Wasserstoff oder Alkyl mit 1, 2, 3 oder 4 C-Atomen;
- R(9): Wasserstoff oder Alkyl mit 1, 2, 3 oder 4 C-Atomen;
- R(10): Wasserstoff oder Alkyl mit 1 oder 2 C-Atomen
- R(11): Phenyl,
wobei Phenyl unsubstituiert ist oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
- R(2): Wasserstoff;
- R(3): Alkyl mit 3, 4 oder 5 C-Atomen, Phenyl,
wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Ebenfalls ganz besonders bevorzugt sind auch Verbindungen der Formel I, worin bedeuten: 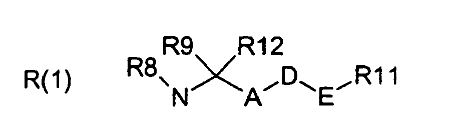
- A: -CₙH₂ₙ-;
n 0, 1 oder 2;
- D: eine Bindung oder Sauerstoff;
- E: -CₘH₂ₘ-;
m 0 oder 1;
- R(8): Wasserstoff oder Alkyl mit 1, 2, 3 oder 4 C-Atomen;
- R(9): Wasserstoff oder Alkyl mit 1, 2, 3 oder 4 C-Atomen;
- R(11): Phenyl oder Pyridyl,
wobei Phenyl und Pyridyl unsubstituiert sind oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(12): Alkyl mit 1, 2, 3 oder 4 C-Atomen oder Cyclopropyl;
- R(2): Wasserstoff;
- R(3): Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Des weiteren ganz besonders bevorzugt sind Verbindungen der Formel I, worin bedeuten: 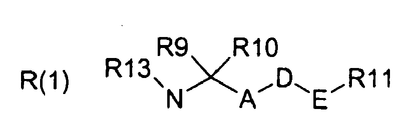
- A: -CₙH₂ₙ-;
n 0, 1 oder 2;
- D: eine Bindung oder Sauerstoff;
-CₘH₂ₘ-;
m = 0 oder 1;
- R(9): Wasserstoff oder Alkyl mit 1 oder 2 C-Atomen;
- R(10): Wasserstoff oder Alkyl mit 1 oder 2 C-Atomen;
- R(11): Cycloalkyl mit 3, 4, 5 oder 6 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
- R(13): CpH₂p-R(14');
p 0, 1, 2, 3 oder 4;
- R(14'): Aryl oder Heteroaryl, wobei Aryl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff;
- R(3): Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

Alkylreste und Alkylenreste können geradkettig oder verzweigt sein. Dies gilt auch für die Alkylenreste der Formeln CₙH₂ₙ, CₘH₂ₘ und CₚH₂ₚ. Alkylreste und Alkylenreste können auch geradkettig oder verzweigt sein, wenn sie substituiert sind oder in anderen Resten enthalten sind, z. B. in einem Alkoxyrest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,1-Butylen, 1,4-Butylen, 1,5-Pentylen, 2,2-Dimethyl-1,3-propylen, 1,6-Hexylen, usw. sind Beispiele für Alkylenreste.

Cycloalkylreste können ebenfalls verzweigt sein. Beispiele für Cycloalkylreste mit 3 bis 7 C-Atomen sind Cyclopropyl, Cyclobutyl, 1-Methylcyclopropyl, 2-Methylcyclopropyl, Cyclopentyl, 2-Methylcyclobutyl, 3-Methylcyclobutyl, Cyclopentyl, Cyclohexyl, 2-Methylcyclohexyl, 3-Methylcyclohexyl, 4-Methylcyclohexyl, Cycloheptyl usw.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also z.B. 1-Oxy-2-, 3- oder 4-pyridyl. Besonders bevorzugt sind die Heteroaromaten Thienyl, Furyl, Pyrrolyl, Imidazolyl, Chinolyl, Pyrazolyl, Pyridyl, Pyrazinyl, Pyrimidinyl und Pyridazinyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Aryl steht für Phenyl und 2- oder 3-Naphthyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position, trisubstituierte in der 2,3,4-, 2,3,5-, 2,3,6-, 2,4,5-, 2,4,6- oder 3,4,5-Position. Entsprechendes gilt sinngemäß analog auch für die N-haltigen Heteroaromaten, den Naphthyl, Thiophen- oder den Furylrest.

Bei Di- bzw. Trisubstitution eines Restes können die Substituenten gleich oder verschieden sein.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Bei Verbindungen der Formel I, bei denen R2 Wasserstoff bedeutet, ist z.B. eine Deprotonierung der Sulfonamidfunktion unter Bildung eines Natriumsalzes möglich. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel 1 gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfasst die vorliegende Erfindung auch alle tautomeren Formen der Verbindungen der Formel I.

Die Verbindungen der Formel I sind durch unterschiedliche chemische Verfahren herstellbar, von denen einige Beispiele unten als Schema 1 oder 2 skizziert sind. Die hierbei verwendeten Reste R(1 ) bis R(7) sind jeweils wie oben angegeben definiert.

Gemäß Schema 1 können erfindungsgemäße Verbindungen beispielsweise hergestellt werden, indem zunächst eine Aminocarbonsäure der Formel II in einem Lösungsmittel wie Wasser, Pyridin oder einem Ether in Gegenwart einer Base mit einem Sulfonylchlorid der Formel R(3)-SO₂-Cl oder einem Sulfonsäureanhydrid umgesetzt wird. Als Base kommen anorganische Basen wie zum Beispiel Natriumcarbonat oder Kaliumhydroxid oder organische Basen wie zum Beispiel Pyridin oder Triethylamin in Betracht.

Die erhaltene Sulfonylaminocarbonsäure der Formel III kann dann zum Beispiel durch Umsetzung mit einem Chlorierungsagenz wie zum Beispiel Phosphorpentachlorid, Phosphoroxychlorid oder Thionylchlorid in einem inerten Lösungsmittel zu einem Säurechlorid aktiviert werden und dann mit einem Amin der Formel H-R1 zu den Titelverbindungen der Formel I umgesetzt. werden. Die Aktivierung der Carbonsäuregruppe in der Verbindung der Formel III kann aber auch auf andere Art erfolgen, zum Beispiel durch eine der zahlreichen, dem Fachmann geläufigen Methoden, die in der Peptidchemie zur Knüpfung von Amidbindungen angewandt werden, zum Beispiel durch Überführung in ein gemischtes Anhydrid oder einen aktivierten Ester oder unter Verwendung eines Carbodiimids wie Dicyclohexylcarbodümid.

Die Umsetzung der aktivierten Sulfonylaminocarbonsäure mit einem Amin der Formel H-R1 wird vorteilhaft in einem inerten Lösungsmittel wie zum Beispiel Pyridin, Tetrahydrofuran oder Toluol ohne Zusatz oder unter Zusatz einer inerten Hilfsbase, zum Beispiel eines tertiären Amins oder von Pyridin, durchgeführt.

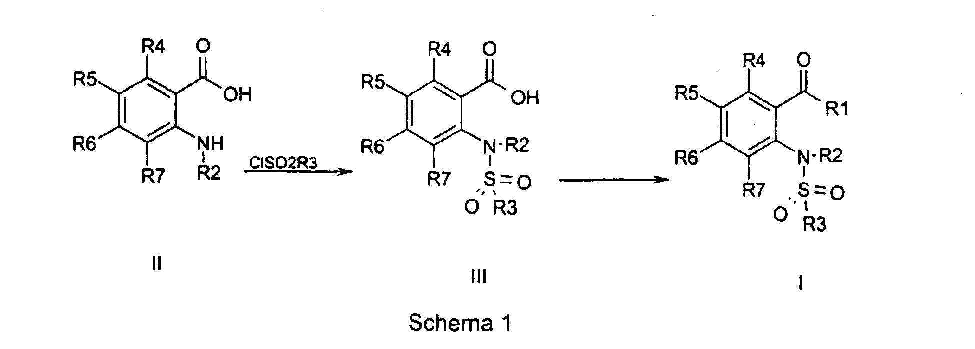

Alternativ können gemäß Schema 2 auch zunächst die Anhydride der Formel IV mit einem Amin der Formel H-R1 zu einem o-Aminobenzamid der Formel VII umgesetzt werden, aus dem dann durch Umsetzung mit einem Sulfonsäurechlorid der Formel R(3)SO₂Cl eine Verbindung der Formel I erhalten wird. Eine andere Möglichkeit zur Herstellung von Zwischenprodukten der Formel VII, bei denen R(2) Wasserstoff bedeutet, besteht in der Amidierung einer o-Nitrobenzoesäure der Formel V mit einem Amin der Formel HNR(1 )R(2) gefolgt von Reduktion der Nitrogruppe zum Amin.

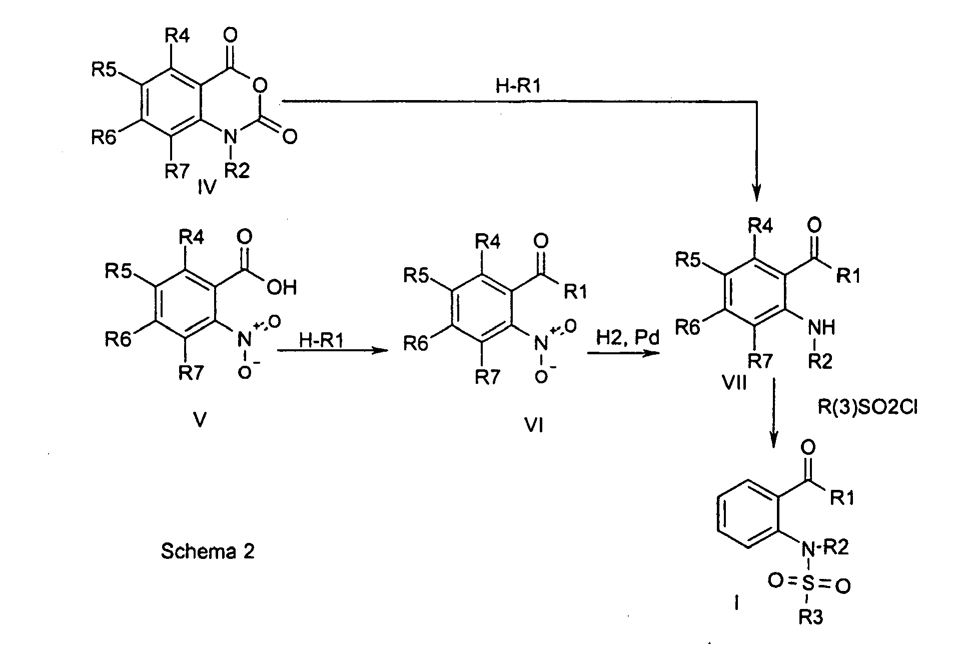

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse 1-, Klasse II- oder Klasse III-Antiarrhythmika, in Frage, wie beispielsweise IKₛ- oder IKᵣ-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wässrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- DMAP: 4-Dimethylaminopyridin
- DMF: N,N-Dimethylformamid
- EDAC: N-Ethyl-N'-(3-dimethylaminopropyl)-carbodiimid Hydrochlorid
- EE: Essigsäureethylester
- HOBT: 1-Hydroxy-1H-benzotriazol
- RT: Raumtemperatur
- THF: Tetrahydrofuran
- BuLi: Butyllithium

### Allgemeine Vorschrift 1: Umsetzung von Anthranilsäuren mit Sulfonylchloriden zu o-Sulfonylaminobenzoesäuren (analog Organic Syntheses 1952, 32, 8)

Zu einer Lösung von 260 g (2,4 mol) Natriumcarbonat und 1 mol der entsprechenden Anthranilsäure in 1,5 l Wasser werden bei 60°C 1,2 mol des entsprechenden Sulfonsäurechlorids portionsweise zugegeben. Die Reaktionsmischung wird bis zur vollständigen Umsetzung (ca. 1 - 6 h) bei 60 - 80°C erhitzt, wobei falls erforderlich weiteres Sulfonsäurechlorid nachgesetzt wird. Nach dem Abkühlen wird die Reaktionsmischung in 500 ml 6 molare Salzsäure gegossen und der ausgefallene Niederschlag wird abgesaugt und im Trockenschrank bei 45°C im Vakuum getrocknet. Falls das Produkt nicht kristallin anfällt wird es durch Extraktion mit Essigsäureethylester isoliert.

Vorstufe 1 a: 2-(Toluol-4-sulfonylamino)-benzoesäure

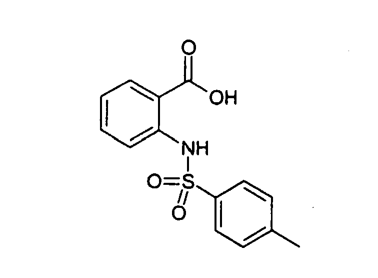

Gemäß der allgemeinen Vorschrift 1 wurden aus 6,85 g Anthranilsäure und 10,5 g para-Toluolsulfonsäurechlorid 9,6 g der Titelverbindung als weisser Feststoff erhalten. MS (ES): 293 (M+1).

Vorstufe 1 b: 2-Butylsulfonylamino-5-methylbenzoesäure

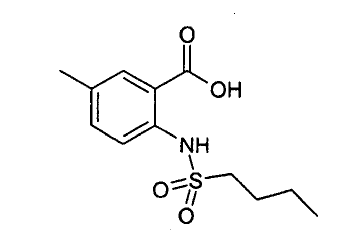

Gemäß der allgemeinen Vorschrift 1 wurden aus 5 g 5-Methylanthranilsäure und 6,2 g Butansulfonylchlorid 4,2 g 2-Butylsulfonylamino-5-methylbenzoesäure erhalten.
MS (ES): 272 (M+1).

Vorstufe 1 c: 2-(4-Methoxybenzolsulfonylamino)-6-methyl-benzoesäure

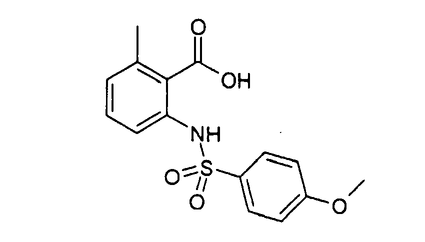

Gemäß der allgemeinen Vorschrift 1 wurden aus 1,5 g 6-Methylanthranilsäure und 2,3 g 4-Methoxybenzolsulfonylchlorid 1,6 g der Titelverbindung als zähes Öl erhalten. MS (ES): 323 (M+1).

Gemäss der allgemeinen Vorschrift 1 wurden unter anderem folgende weitere Vorstufen synthetisiert:

| Vorstufe | Struktur | Masse (ES) |
|---|---|---|
| 1 d | 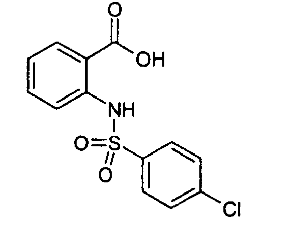 | 312 (M+1) |
| 1 e | 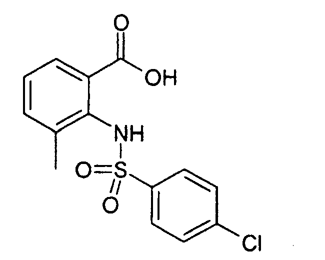 | 326 (M+1) |
| 1 f | 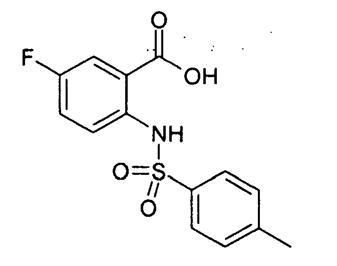 | 310 (M+1) |
| 1 g | 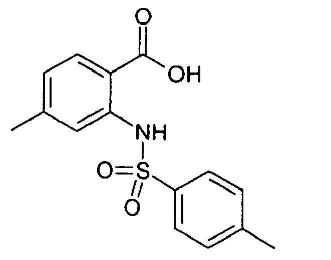 | 306 (M+1) |
| 1 h | 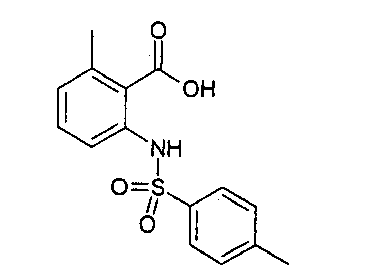 | 306 (M+1) |
| 1 i | 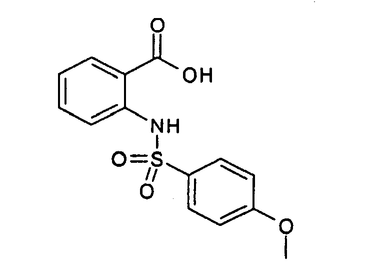 | 308 (M+1) |
| 1 j | 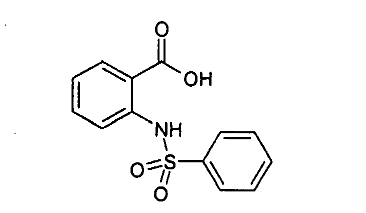 | 278 (M+1) |
| 1 k | 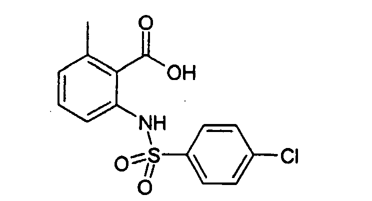 | 326 (M+1) |
| 1 l | 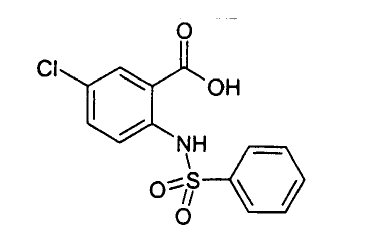 | 312 (M+1) |
| 1 m | 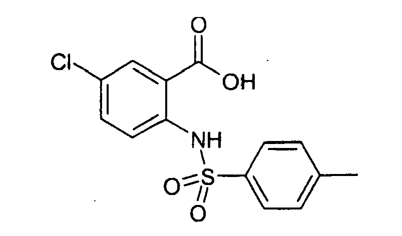 | 326 (M+1) |
| 1 n | 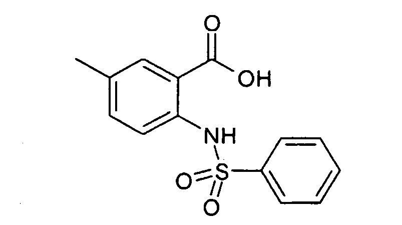 | 292 (M+1) |
| 1 o | 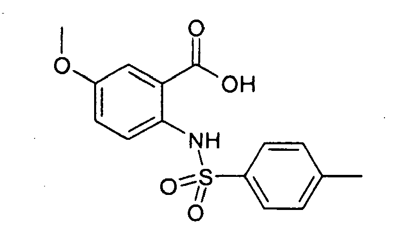 | 322 (M+1) |
| 1 p | 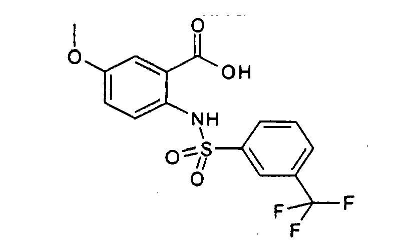 | 376 (M+1) |
| 1 q | 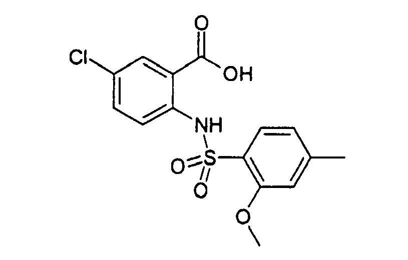 | 356 (M+1) |
| 1 r | 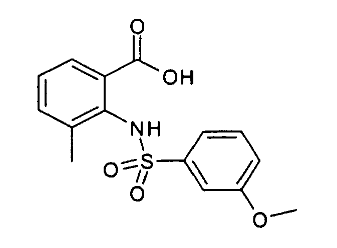 | 322 (M+1) |
| 1 s | 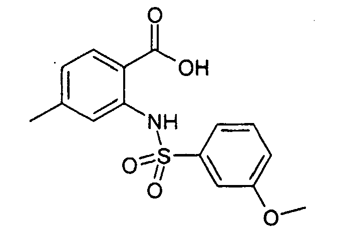 | 322 (M+1) |
| 1 t | 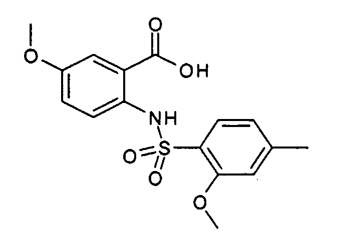 | 352 (M+1) |
| 1 u | 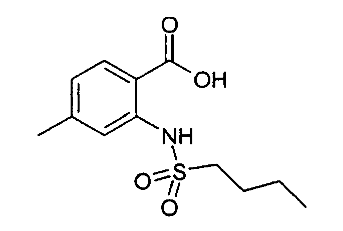 | 272 (M+1) |

### Allgemeine Vorschrift 2: Umsetzung von Sulfonylaminobenzoesäuren zu den entsprechenden Säurechloriden

### A) mit Phosphorpentachlorid

8 mmol der Sulfonylaminobenzoesäure werden in 15 ml trockenem Toluol suspendiert und bei Raumtemperatur langsam 9,6 mmol Phosphorpentachlorid eingetragen. Die Mischung wird 3h bei 50°C gerührt, auf 0°C abgekühlt, das Säurechlorid abgesaugt, mit wenig Toluol gewaschen und bei 45°C im Vakuumtrockenschrank getrocknet.

Vorstufe 2 a: 2-(4-Toluolsulfonylamino)-benzoylchlorid

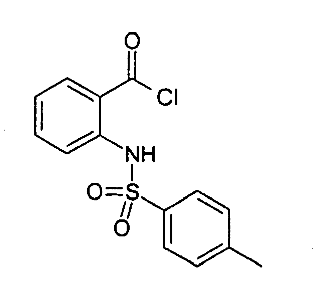

Aus 9,6 g 2-(Toluol-4-sulfonylamino)-benzoesäure (Vorstufe 1 a) und 8,3 g Phosphorpentachlorid wurden 7,5 g der Titelverbindung als weisser Feststoff isoliert. MS (ES, als Methylester nach Zugabe von Methanol detektiert): 306 (M+1).

### B) mit Thionylchlorid

8 mmol der Sulfonylaminobenzoesäure werden in 6 ml Thionylchlorid 3 h auf 60°C erhitzt, eingeengt und der Rückstand zweifach mit Toluol koevaporiert.

Vorstufe 2 b: 2-(4-Methoxybenzolsulfonylamino)-benzoesäurechlorid

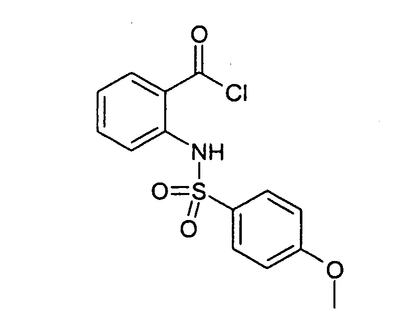

Aus 2,4 g 2-(4-Methoxybenzolsulfonylamino)-benzoesäure und 5 ml Thionylchlorid wurden 2,2 g der Titelverbindung erhalten.
MS (ES, als Methylester nach Zugabe von Methanol detektiert): 322 (M+1).

Gemäss der allgemeinen Vorschrift 2 (Variante A oder B) wurden unter anderem folgende weitere Vorstufen hergestellt:

| Vorstufe | Struktur | Masse (ES nach Zugabe von Methanol zum Säurechlorid) Detektion der Methylester |
|---|---|---|
| 2c | 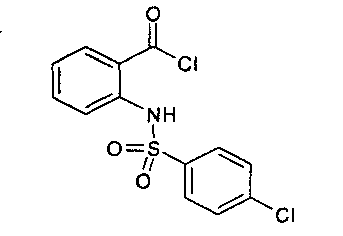 | 326 (M+1) |
| 2 d | 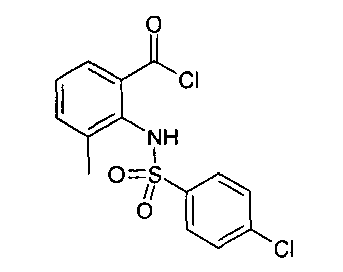 | 340 (M+1) |
| 2e | 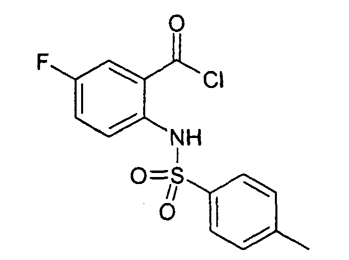 | 324 (M+1) |
| 2 f | 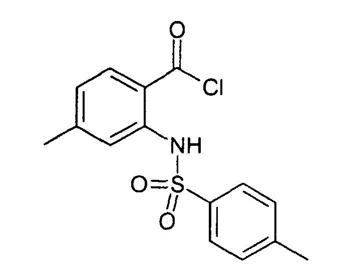 | 320 (M+1) |
| 2 g | 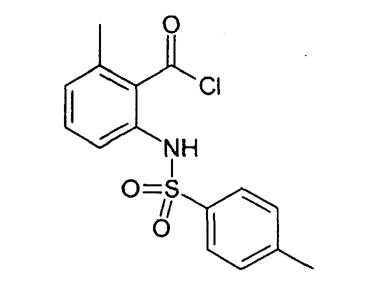 | 320 (M+1) |
| 2 h | 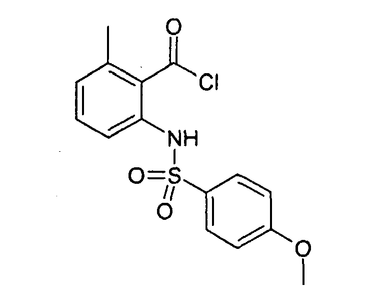 | 336 (M+1) |
| 2 i | 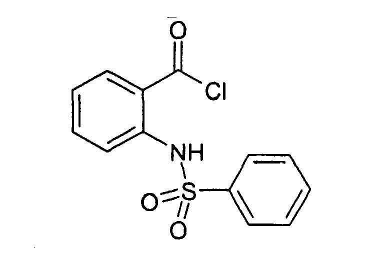 | 292 (M+1) |
| 2 j | 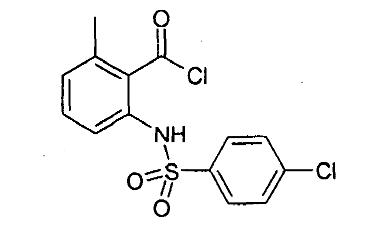 | 340 (M+1) |
| 2 k | 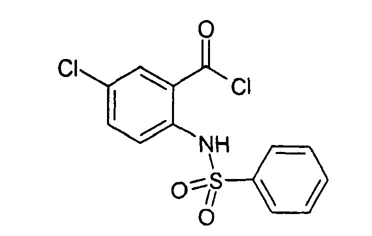 | 326 (M+1) |
| 2 l | 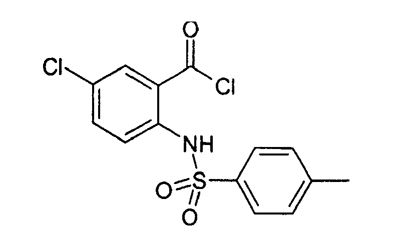 | 340 (M+1) |
| 2 m | 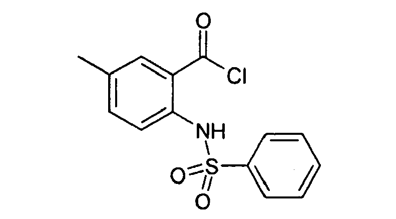 | 306 (M+1) |
| 2 n | 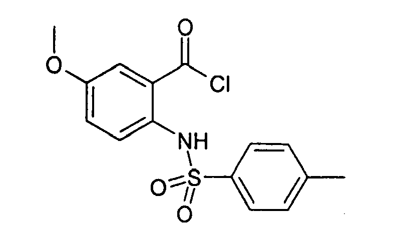 | 336 (M+1) |
| 2 o | 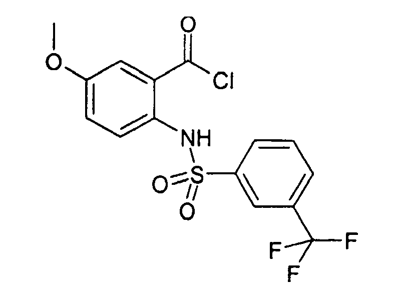 | 390 (M+1) |
| 2 p | 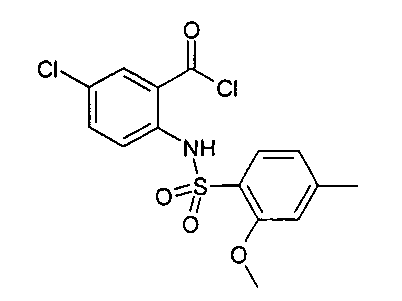 | 370 (M+1) |
| 2 q | 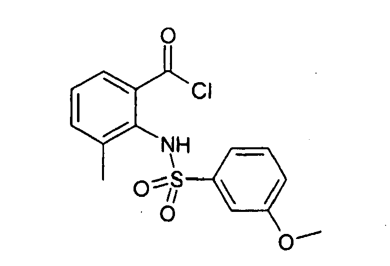 | 336 (M+1) |
| 2 r | 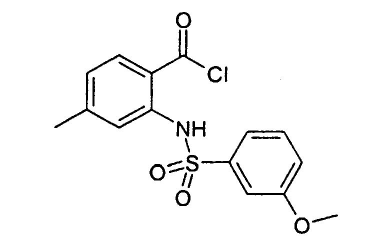 | 336(M+1 ) |
| 2 s | 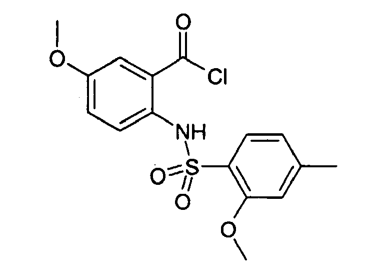 | 366 (M+1) |
| 2 t | 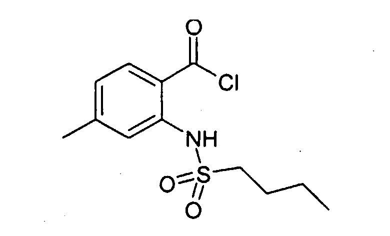 | 286 (M+1) |
| 2 u | 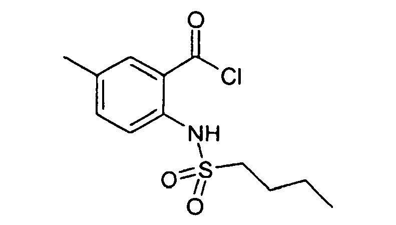 | 286 (M+1) |

Allgemeine Vorschrift 3 A: Darstellung sekundärer Amine durch reduktive Aminierung 0,18 mmol primäres Amin werden in 200 ml Methanol gelöst, mit 0,09 mol Aldehyd, 0,18 mmol Natriumcyanborhydrid sowie 0,18 mmol Eisessig versetzt und 6h bei Raumtemperatur gerührt. Die Lösung wird eingeengt, mit Essigester aufgenommen und zweimal mit NaHCO₃-Lösung gewaschen. Die organische Phase wird eingeengt und der Rückstand im Hochvakuum destilliert. Bei schwer flüchtigen sekundären Aminen werden flüchtige Bestandteile abdestilliert und der Rückstand in Ether/THF gelöst und mit etherischer HCl-Lösung versetzt und das ausgefallene Hydrochlorid abgesaugt, mit Ether gewaschen und getrocknet. Die hergestellten sekundären Amine wurde ohne weitere Reinigung für die Umsetzungen mit den Sulfonylaminobenzoylchloriden oder Sulfonylaminobenzoesäuren eingesetzt.

Vorstufe 3 a: Benzyl-(1-methyl-1H-imidazol-2-ylmethyl)-amin

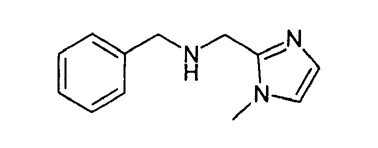

Gemäss allgemeiner Arbeitsvorschrift wurde aus 19,4 g Benzylamin und 10 g 2-Formyl-1-methylimidazol das Hydrochlorid (20,5 g) hergestellt.
MS (ES+): m/z = 202 (M+1).

Vorstufe 3 b: Benzyl-pyridin-3-ylmethylamin

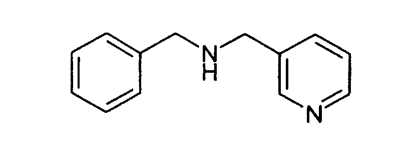

Gemäss allgemeiner Arbeitsvorschrift wurde aus 4,32 g 3-Pyridylmethylamin und 2,12 g Benzaldehyd nach Kugelrohrdestillation bei 0,1 mbar und 130°C das sekundäre Amin (2,8 g) hergestellt.
MS (ES+): m/z = 199 (M+1).

Vorstufe 3 c: Benzyl-(3-imidazol-1-yl-propyl)-amin

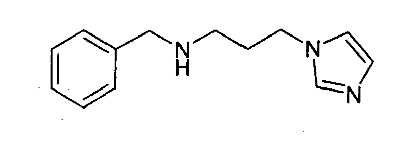

Gemäss allgemeiner Arbeitsvorschrift wurden aus 12.5 g 3-Imidazol-1-yl-propylamin und 5.3 g Benzaldehyd nach Kugelrohrdestillation bei 0.1 mbar und 130°C das sekundäre Amin (3.5 g) hergestellt.
MS (ES+): m/z = 216 (M+1).

Gemäss der allgemeinen Arbeitsvorschrift 3A wurden unter anderem folgende weitere Vorstufen hergestellt:

| Vorstufe | Struktur | Masse |
|---|---|---|
| 3 d | 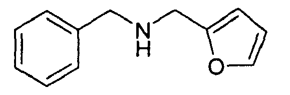 | 188 (M+1) |
| 3 e | 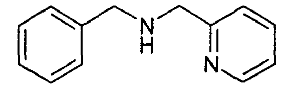 | 199 (M+1) |
| 3 f | 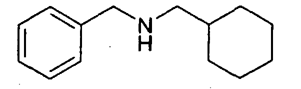 | 204 (M+1) |
| 3 g | 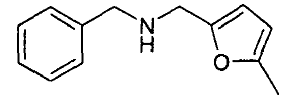 | 202 (M+1) |
| 3 h | 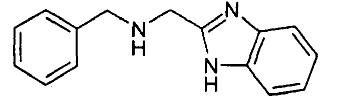 | 238 (M+1) |
| 3 i | 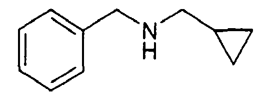 | 162 (M+1) |
| 3 j | 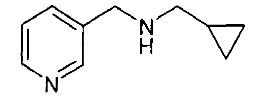 | 163 (M+1) |
| 3k | 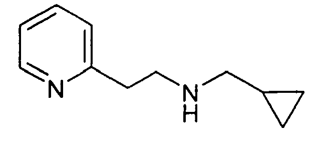 | 177 (M+1) |
| 3o | 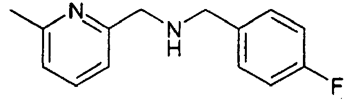 | 231 (M+1) |
| 3p | 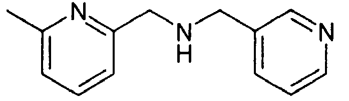 | 214 (M+1) |
| 3q | 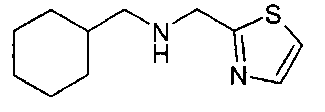 | 211 (M+1). |
| 3r | 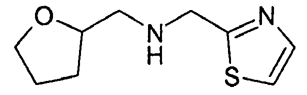 | 199 (M+1) |

### Allgemeine Vorschrift 3 B: Darstellung von α-verzweigten Aminen aus Ketonen

Zu einer Lösung von 200 mmol Hydroxylammoniumchlorid und 200 ml Natriumacetat in 120 ml Wasser wird bei 30°C eine Lösung von 67 mmol des entsprechenden Ketons in 120 ml Ethanol zugetropft und es wird bis zur vollständigen Umsetzung (1 - 3 h) auf 60°C erhitzt. Nach dem Abkühlen wird die Reaktionsmischung mit Wasser verdünnt und das ausgefallene Oxim wird abgesaugt oder falls erforderlich durch Extraktion isoliert. Das erhaltene Produkt wird in 100 ml Methanol, 100 ml THF und 10 ml konzentrierter Ammoniaklösung gelöst und in Gegenwart von Raney-Nickel bei RT und Normaldruck bis zum Ende der Wasserstoffaufnahme hydriert. Nach Abfiltrieren des Katalysators und Einengen der Reaktionsmischung erhält man das entsprechende Amin, das falls erforderlich, chromatographisch gereinigt wird.

Vorstufe 3 1: 1-Benzyl-propylamin

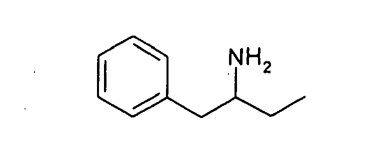

Gemäß der allgemeinen Vorschrift 3 B wurden aus 10 g 1-Phenyl-2-butanon 4,5 g der Titelverbindung erhalten.

Vorstufe 3 m: 1-Pyridin-4-yl-propylamin

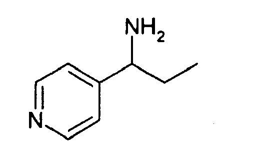

Gemäß der allgemeinen Vorschrift 3 B wurden aus 10 g 4-Propionylpyridin 10,2 g der Titelverbindung erhalten.

Vorstufe 3 n : 1-Pyridin-3-yl-propylamin

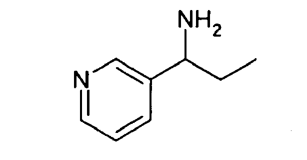

Gemäß der allgemeinen Vorschrift 3 B wurden aus 1 g 4-Propionylpyridin 0,9 g der Titelverbindung erhalten.

Vorstufe 3s: 1-Cyclopropyl-1-phenylmethylamin Hydrochlorid

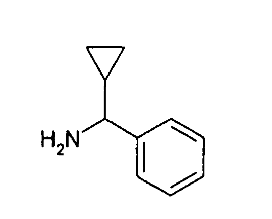

### a) N-(Cyclopropylphenylmethyl)-formamid

14,8 g (0,1 mol) Cyclopropylphenylketon, 11,4 ml (0,3 mol) Ameisensäure und 20 ml (0,5 mol) Formamid wurden 18 h auf 160°C erhitzt. Nach dem Abkühlen wurde mit 100 ml Wasser versetzt und 2x mit je 50 ml Ether extrahiert. Die etherische Phase wurde mit 50 ml 10 % Na₂CO₃ - Lösung gewaschen, über Na₂SO₄ getrocknet und eingeengt. Man erhielt 13,6 g (77,4 mmol) eines gelben Öls.

### b) 1-Cyclopropyl-1-phenylmethylamin Hydrochlorid

13,6 g (77,4 mmol) N-(Cyclopropylphenylmethyl)-formamid (siehe a) wurden 18 h in 100 ml 2N HCl zum Rückfluss erhitzt. Nach dem Abkühlen wurde 2x mit je 50 ml Dichlormethan extrahiert und die wässrige Phase eingeengt. Der Rückstand wurde in 30 ml 2-Propanol aufgenommen, zum Sieden erhitzt und über Nacht im Kühlschrank abgekühlt. Die ausgefallenen Kristalle an 1-Cyclopropyl-1-phenylmethylamin Hydrochlorid (3,85 g, 21 mmol) wurden abgesaugt und im Vakuumtrockenschrank getrocknet.

Vorstufe 3t: Cyclopropylpyridin-2-yl-methylamin Hydrochlorid

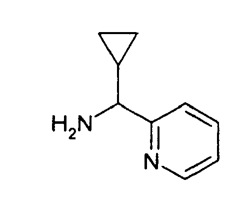

### a) Cyclopropylpyridin-2-yl-methylenamin

Zu 100 ml (160 mmol) n-BuLi-Lösung in 300 ml Diethylether wurden bei -70°C 25 g (157,5 mmol) 2-Brompyridin in 100 ml Diethylether innerhalb von 20 min zugetropft. Die dunkelrote Lösung wurde 5 h gerührt und dann mit 8,8 g (131 mmol) Cyclopropancarbonsäurenitril in 100 ml Ether versetzt. Die Mischung wurde bei -70°C 30 min gerührt, auf Raumtemperatur erwärmt und weitere 30 min gerührt. Anschliessend wurden 15 g Na₂SO₄ x 10 H₂0 zugesetzt und 1 h weitergerührt. Die rote Lösung wurde mit Na₂SO₄ versetzt, abfiltriert und eingeengt. In der Kugelrohrdestille wurde das Produkt bei 75°C - 120°C /0.3 mbar als hellgelbes Öl (18,6 g, 127 mmol) destilliert und bei -18°C aufbewahrt.

### b) Cyclopropylpyridin-2-yl-methylamin Hydrochlorid

2,72 g (18,6 mmol) Cyclopropylpyridin-2-yl-methylenamin (siehe a) wurden in 35 ml trockenem Methanol gelöst. Bei 0°C wurde portionsweise 0,69 g (18,6 mmol) NaBH₄ zugesetzt. Nach 30 min bei 0°C wurde 2 h bei Raumtemperatur gerührt, mit 1 M HCl auf pH 3 gestellt, das Methanol am Rotationsverdampfer abgezogen und der Rückstand gefriergetrocknet. Man erhielt 8,8 g Cyclopropylpyridin-2-yl-methylamin Hydrochlorid das mit anorganischen Salzen und Borsäure vermischt ist.

Vorstufe 3 u: Cyclopropylpyridin-3-yl-methylamin Hydrochlorid

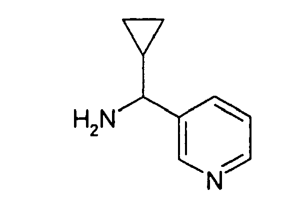

### a) Cyclopropylpyridin-3-yl-methylenamin

Entsprechend der Vorschrift für Vorstufe 3p wurden aus 8,8 g (131 mmol) Cyclopropancarbonsäurenitril, 25 g (157,5 mmol) 3-Brompyridin und 173 mmol n-BuLi-Lösung nach Kugelrohrdestillation (130°C/0,2 mbar) 7,5 g (51 mmol) des Imins als gelbes Öl isoliert.

### b) Cyclopropylpyridin-3-yl-methylamin Hydrochlorid

Entsprechend der Vorschrift für Vorstufe 3p wurden aus 7,5 g (51,5 mmol) Imin (siehe a) und 1,9 g (51,4 mmol) NaBH₄ 16,6 g Cyclopropylpyridin-3-yl-methylamin Hydrochlorid erhalten die mit anorganischen Salzen und Borsäure vermischt ist.

Vorstufe 3 v: 1-(5-Methyl-furan-2-yl)-propylamin

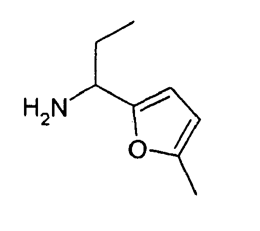

Zu 5 g (36 mmol) 2-Methyl-5-propionylfuran und 28,2 g (366 mmol) Ammoniumacetat in 300 ml Methanol wurde portionsweise 11,35 g (180 mmol) Natriumcyanborhydrid unter Rühren eingetragen und 18 h bei RT reagieren gelassen. Die Mischung wurde weitgehend eingeengt, mit 200 ml Dichloromethan versetzt und die organische Phase 3x mit je 50 ml NaHCO₃-Lösung gewaschen, über Na₂SO₄ getrocknet und eingeengt. Man erhielt 3,9 g (28 mmol) des Amins in Form eines hellgelben Öls.

Vorstufe 3w: 1-Phenyl-prop-2-ynylamin Hydrochlorid

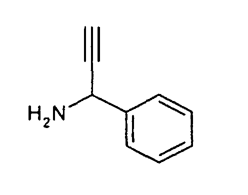

Die Verbindung wurde entsprechend der Vorschrift von Bjorn M. Nilsson et al, J. Heterocycl. Chem. (1989), 26(2), 269-75 ausgehend von 1-Phenyl-2-propynylalkohol durch Ritter-Reaktion und anschliessende salzsaure Hydrolyse hergestellt.

Vorstufe 3x: C-Cyclopropyl-C-(6-methoxy-pyridin-2-yl)-methylamin

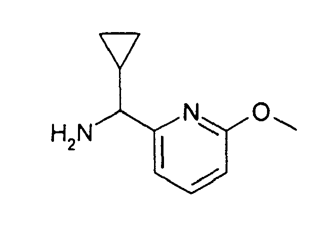

### a) Cyclopropancarbaldehyd- O-benzyloxim

6,7 g (95,6 mmol) Cyclopropancarbaldehyd wurde zusammen mit 15,3 g (95,6 mmol) O-Benzylhydroxylamin und 15,7 g (191,2 mmol) Natriumacetat in 250 ml Ethanol bei Raumtemperatur 18h gerührt, eingeengt und mit Na₂SO₄ versetzt. Der Rückstand wurde 3x mit je 50 ml Dichloromethan extrahiert, die organische Phase eingeengt und das Rohprodukt an Kieselgel chromatographisch gereinigt. Man erhielt 5 g (28,6 mmol) einer farblosen Flüssigkeit.

### b) O-Benzyl-N-[cyclopropyl-(6-methoxypyridin-2-yl)-methyl]-hydroxylamin

3,76 g (20 mmol) 2-Bromo-6-methoxypyridin wurden in 20 ml THF bei -78°C mit 8,8 ml (22 mmol) n-BuLi (2,5 M in Toluol) versetzt. Nach 30 min wurde diese dunkelrote Lösung zu einer Lösung von 1,4 g (8 mmol) Cyclopropancarbaldehyd- O-benzyloxim (siehe a) und 2,52 ml (20 mmol) BF₃-Etherat in 40 ml Toluol, die bei -78°C 15 min gerührt wurde, zugegeben. Es wurde 4 h bei -78°C gerührt, langsam auf RT erwärmt, mit Wasser versetzt und dann mit gesättigter Na₂CO₃-Lösung alkalisch gestellt. Die organische Phase wurde abgetrennt, die wässrige Phase mit Toluol extrahiert und die vereinigten organischen Phasen über Na₂SO₄ getrocknet und eingeengt. Das Rohprodukt wurde in 12 ml Acetonitril aufgenommen, unlösliche Bestandteile abgetrennt und das Produkt mittels präparativer HPLC isoliert (650 mg, rotes Öl).

### c) C-Cyclopropyl-C-(6-methoxy-pyridin-2-yl)-methylamin

650 mg (2,3 mmol) O-Benzyl-N-[cyclopropyl-(6-methoxypyridin-2-yl)-methyl]-hydroxylamin (siehe b) wurden in 18 ml Eisessig gelöst und mit 18 ml Wasser verdünnt. 3,3 g Zinkstaub wurden dazugegeben und die Suspension 24 h im Ultraschallbad zur Reaktion gebracht Die Mischung wurde über Kieselgur filtriert, mit halbkonzentrierter Essigsäure nachgewaschen, das Filtrat teilweise eingeengt und mit gesättigter Na₂CO₃-Lösung auf pH 11 gebracht. Es wurde 3x mit je 100 ml Dichloromethan extrahiert, über Na₂SO₄ getrocknet und eingeengt. Man erhielt 0,4 g (2,2 mmol) des Produkts in Form eines dunkelroten Öls.

Allgemeine Vorschrift 4 A: Darstellung von 2-Aminobenzoesäureamiden aus 2-Nitrobenzoesäuren

Die entsprechende 2-Nitrobenzoesäure wird zunächst analog der allgemeinen Vorschriften 2 und 5 mit dem jeweiligen Amin zu einem 2-Nitrobenzoesäureamid umgesetzt. Anschließend werden 4 mmol des 2-Nitrobenzoesäureamids in 50 ml THF und 50 ml Methanol in Gegenwart einer Spatelspitze 10 % Palladium auf Kohle bei RT und Normaldruck hydriert. Man saugt vom Katalysator ab, engt die Reaktionsmischung ein und erhält das entsprechende 2-Aminobenzoesäureamid.

Auf diese Weise wurde u.a. folgende Vorstufe synthetisiert:

| Vorstufe | Struktur | Masse |
|---|---|---|
| 4 a | 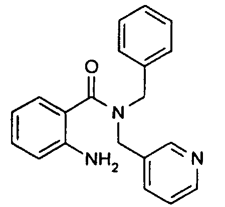 | 318 (M+1) |

### Allgemeine Vorschrift 4 B: Darstellung von 2-Aminobenzoesäureamiden aus Isatosäureanhydrid

Eine Lösung von 20 mmol Isatosäureanhydrid und 22 mmol des entsprechenden Amins in 75 ml DMF wird bis zur vollständigen Umsetzung auf 60°C erhitzt. Die Reaktionsmischung wird mit 100 ml Wasser versetzt und das Produkt wird abgesaugt oder durch Extraktion isoliert.

Vorstufe 4 b : (S)-2-Amino-N-(1-phenyl-propyl)-benzamid

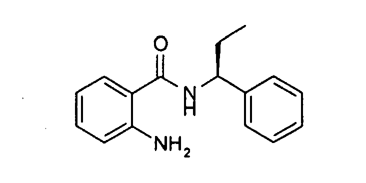

Gemäß der allgemeinen Vorschrift 4 b wurden aus 3 g (S)-1-Phenylpropylamin und 3,2 g Isatoanhydrid nach 2 h bei 60°C 3,4 g der Titelverbindung erhalten.

### Allgemeine Vorschrift 5: Umsetzung von Sulfonylaminobenzoylchloriden mit Aminen

Zu einer Lösung von 0,66 mmol des jeweiligen Amins und 0,9 mmol Triethylamin in 3 ml Methylenchlorid werden 0,6 mmol des jeweiligen Sulfonylaminobenzoylchlorids hinzugefügt und der Ansatz wird über Nacht bei Raumtemperatur gerührt. Die Reaktionsmischung wird mit 5 ml Wasser und 10 ml Methylenchlorid verdünnt und die organische Phase wird nacheinander mit 1 M Salzsäurelösung und gesättigter Natriumbicarbonatlösung gewaschen. Nach Trocknen über Magnesiumsulfat wird die Lösung im Vakuum eingeengt und das Produkt, falls erforderlich, über präparative HPLC oder Säulenchromatographie gereinigt.

### Beispiel 1: (S)-2-Phenylsulfonylamino-5-chlor-N-(1-phenyl-ethyl)-benzamid

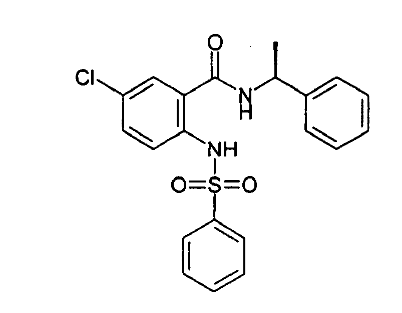

Gemäß der allgemeinen Vorschrift 5 wurden aus 2-Phenylsulfonylamino-5-chlorbenzoylchlorid und S-(-)-1-Methyl-benzylamin 61 mg der Titelverbindung erhalten. MS (ES+): m/z = 415 (M+1).

### Beispiel 2: (R)-2-Phenylsulfonylamino-5-chlor-N-(1-phenyl-ethyl)-benzamid

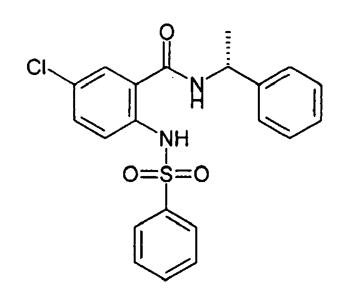

Gemäß der allgemeinen Vorschrift 5 wurden aus 2-Phenylsulfonylamino-5-chlorbenzoylchlorid und R-(+)-1-Methyl-benzylamin 160 mg der Titelverbindung erhalten. MS (ES+): m/z = 415 (M+1).

### Beispiel 3: (S)-2-Phenylsulfonylamino-5-chlor-N-(1-phenyl-propy)-benzamid

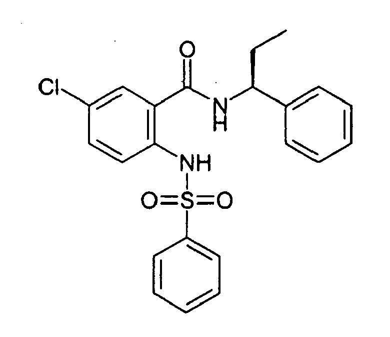

Gemäß der allgemeinen Vorschrift 5 wurden aus 2-Phenylsulfonylamino-5-chlorbenzoylchlorid und S-(-)-1-Ethyl-benzylamin 140 mg der Titelverbindung erhalten. MS (ES+): m/z = 429 (M+1).

### Beispiel 4: (R)-2-Phenylsulfonylamino-5-chlor-N-(1-phenyl-propy)-benzamid

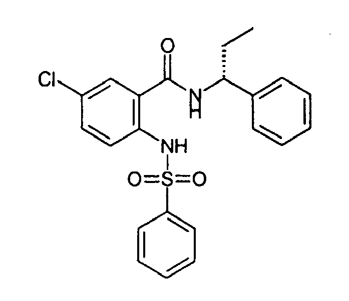

Gemäß der allgemeinen Vorschrift 5 wurden aus 2-Phenylsulfonylamino-5-chlorbenzoylchlorid und R-(+)-1-Ethyl-benzylamin 130 mg der Titelverbindung erhalten. MS (ES+): m/z = 429 (M+1).

### Beispiel 5: (S)-2-Phenylsulfonylamino-5-chlor-N-[1-(4-methoxy-phenyl-ethyl]-benzamid

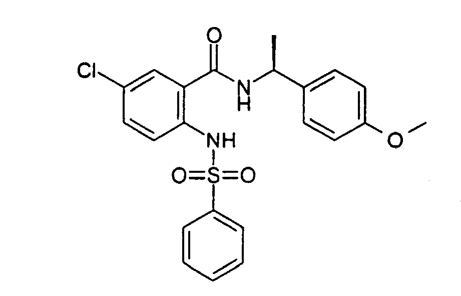

Gemäß der allgemeinen Vorschrift 5 wurden aus 2-Phenylsulfonylamino-5-chlorbenzoylchlorid und S-(-)-1-(4-Methoxy-phenyl)-ethylamin 136 mg der Titelverbindung erhalten. MS (ES+): m/z = 445 (M+1).

### Beispiel 6: (R)-2-Phenylsulfonylamino-5-chlor-N-[1-(4-methoxy-phenyl-ethyl]-benzamid

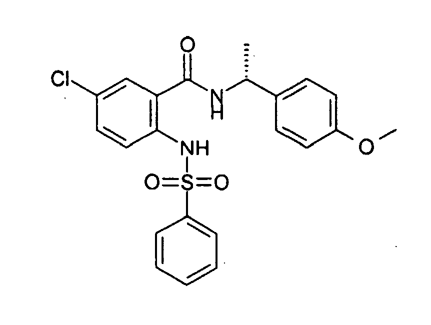

Gemäß der allgemeinen Vorschrift 5 wurden aus 2-Phenylsulfonylamino-5-chlorbenzoylchlorid und R-(+)-1-(4-Methoxy-phenyl)-ethylamin 112 mg der Titelverbindung erhalten. MS (ES+): m/z = 445 (M+1):

### Beispiel 7: 2-Phenylsulfonylamino-5-chloro-N-(phenyl-pyridin-2-yl-methyl)-benzamid

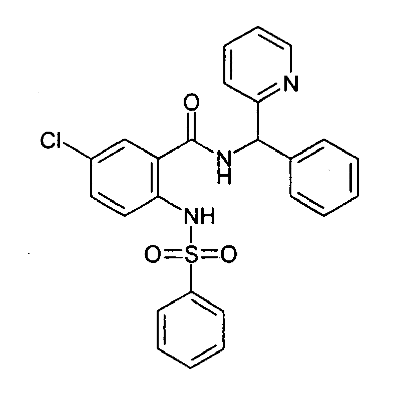

Gemäß der allgemeinen Vorschrift 5 wurden aus 2-Phenylsulfonylamino-5-chlorbenzoylchlorid und C-Phenyl-C-pyridin-2-yl-methylamin 211 mg der Titelverbindung erhalten. MS (ES+): m/z = 478 (M+1).

### Beispiel 8: N-Benzyl-N-pyridin-3-ylmethyl-2-(toluol-4-sulfonylamino)-benzamid

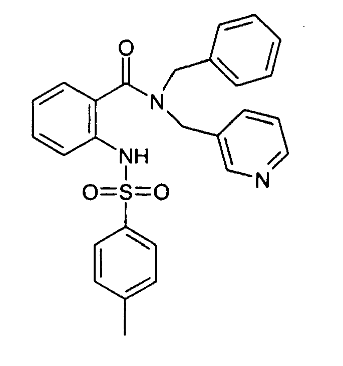

Gemäß der allgemeinen Vorschrift 5 wurden aus 0,93 g 2-para-Toluolsulfonylaminobenzoylchlorid und 0,65 g Benzylpyridin-3-ylmethylamin (Vorstufe 3b) 1,1 g der Titelverbindung erhalten. Die Verbindung wurde nach Zugabe von etherischer HCl-Lösung als weißes Salz isoliert.
MS (ES+): m/z = 472 (M+1).

Entsprechend der allgemeinen Vorschrift 5 wurden u.a. folgende weitere Beispiele hergestellt:

| Beispiel | Struktur | Masse (ES) |
|---|---|---|
| 9 | 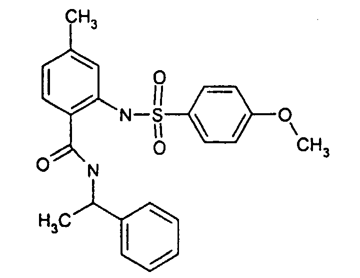 | 425 (M+1) |
| 10 | 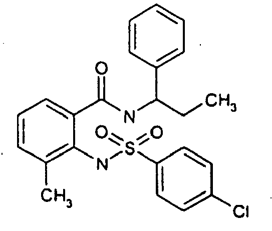 | 443 (M+1) |
| 11 | 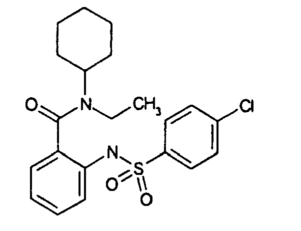 | 421 (M+1) |
| 12 | 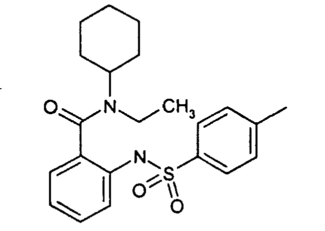 | 401 (M+1) |
| 13 | 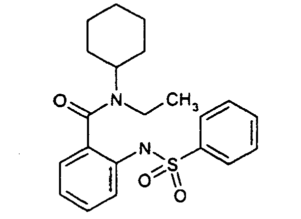 | 387 (M+1) |
| 14 | 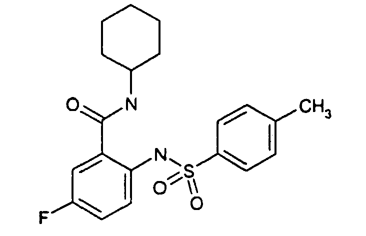 | 391 (M+1) |
| 15 | 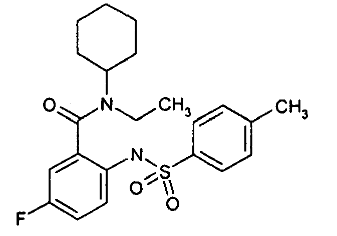 | 419 (M+1) |
| 16 | 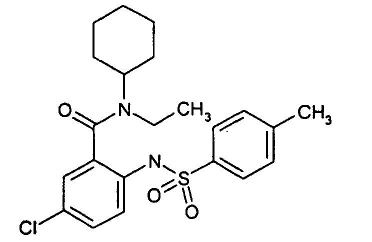 | 435 (M+1) |
| 17 | 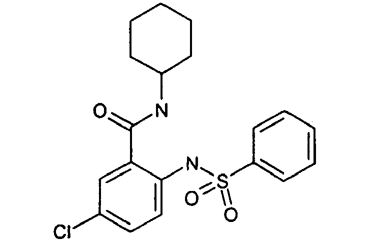 | 393 (M+1) |
| 18 | 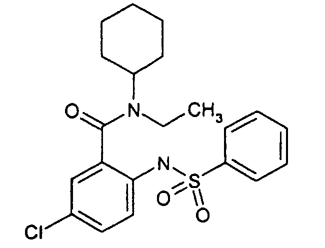 | 421 (M+1) |
| 19 | 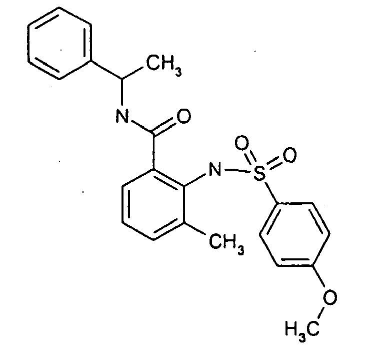 | 425 (M+1) |
| 20 | 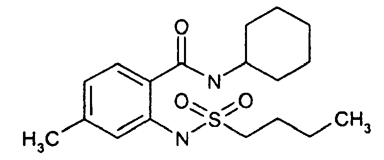 | 353 (M+1) |
| 21 | 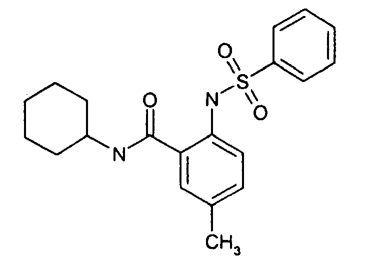 | 373 (M+1) |
| 22 | 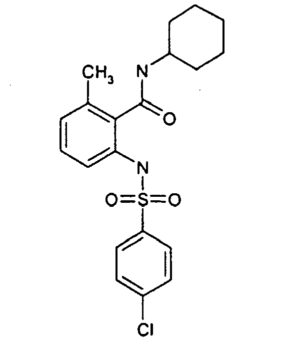 | 407 (M+1) |
| 23 | 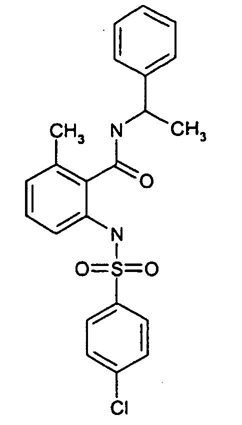 | 429 (M+1) |
| 24 | 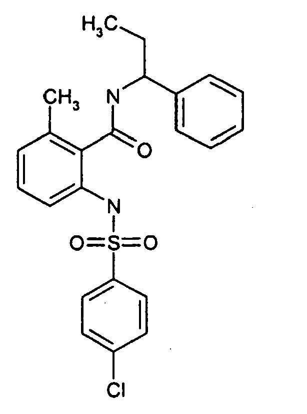 | 443 (M+1) |
| 25 | 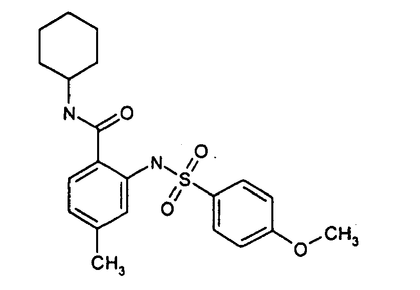 | 403 (M+1) |
| 26 | 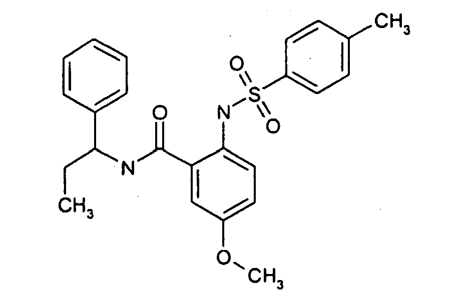 | 439 (M+1) |
| 27 | 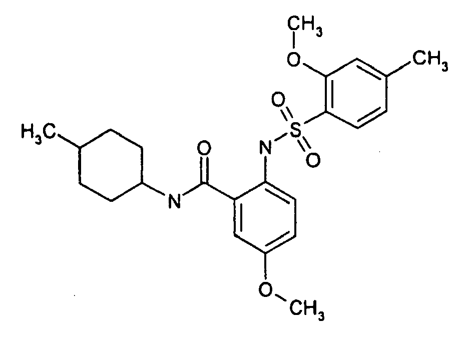 | 447 (M+1) |
| 28 | 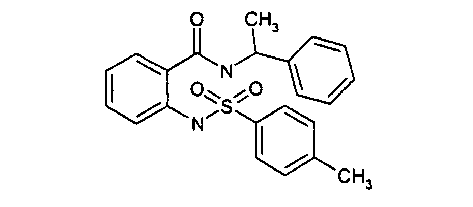 | 395 (M+1) |
| 29 | 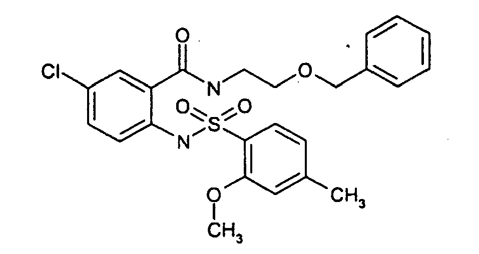 | 489 (M+1) |
| 30 | 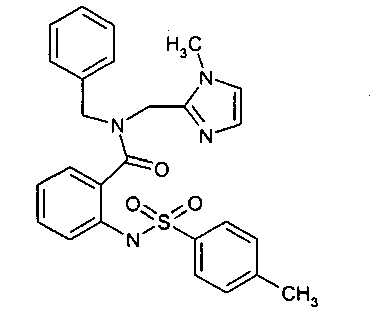 | 475 (M+1) |
| 31 | 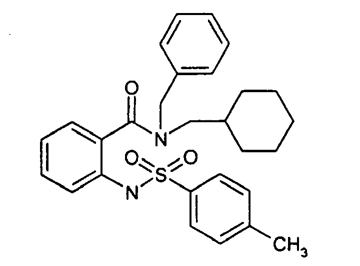 | 477 (M+1) |
| 32 | 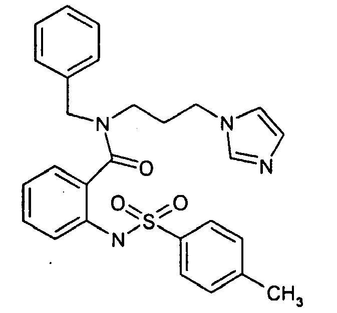 | 489 (M+1) |
| 33 | 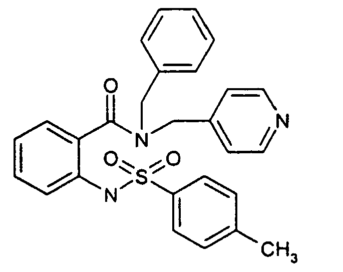 | 472 (M+1) |
| 34 | 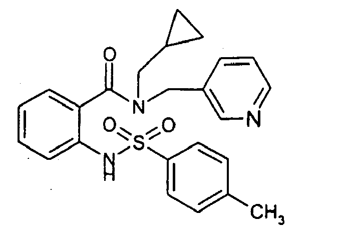 | 436 (M+1) |
| 35 | 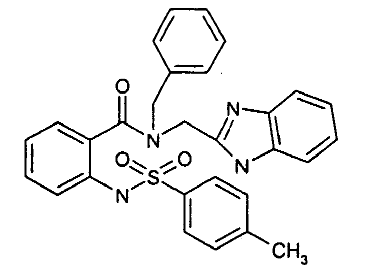 | 511 (M+1) |
| 36 | 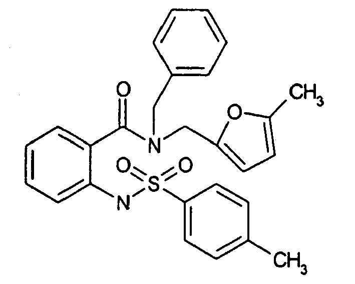 | 475 (M+1) |
| 37 | 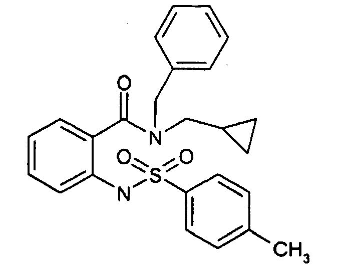 | 435 (M+1) |
| 38 | 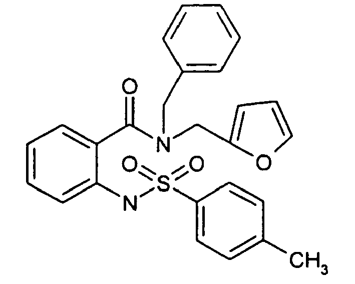 | 461 (M+1) |
| 39 | 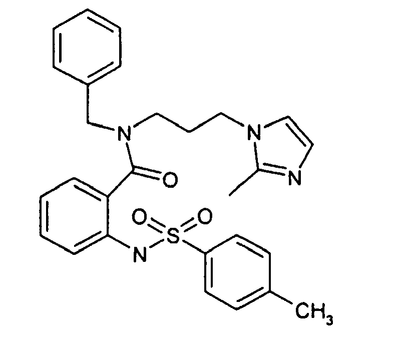 | 503 (M+1) |
| 40 | 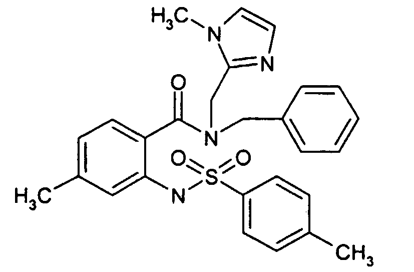 | 489 (M+1) |
| 41 | 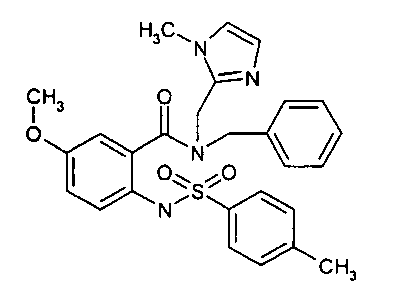 | 505 (M+1) |
| 42 | 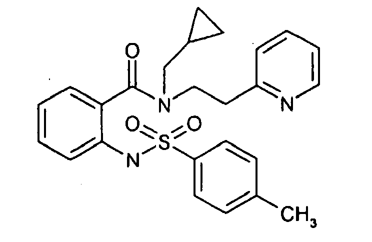 | 450 (M+1) |
| 43 | 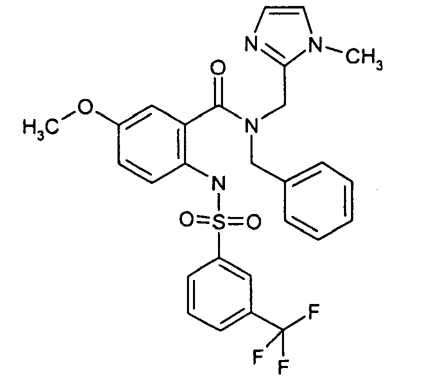 | 559 (M+1) |
| 44 | 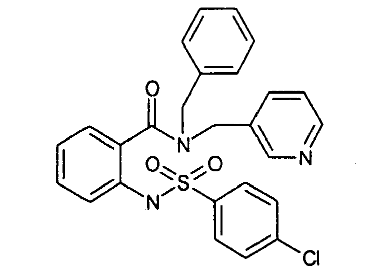 | 492 (M+1) |
| 45 | 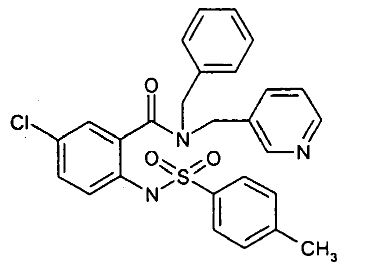 | 506 (M+1) |
| 46 | 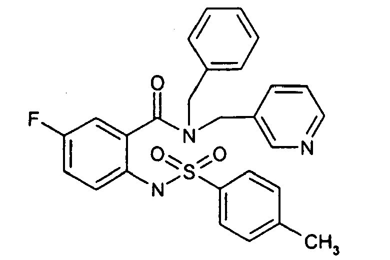 | 490 (M+1) |
| 47 | 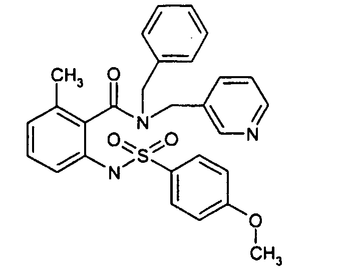 | 502 (M+1) |
| 48 | 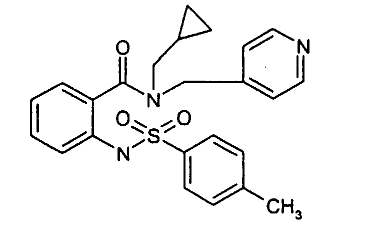 | 436 (M+1) |
| 49 | 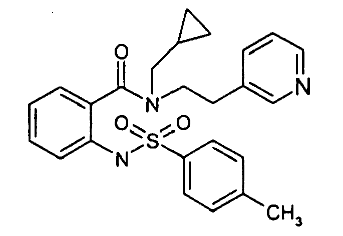 | 450 (M+1) |
| 50 | 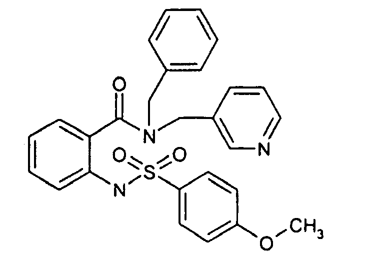 | 488 (M+1) |
| 51 | 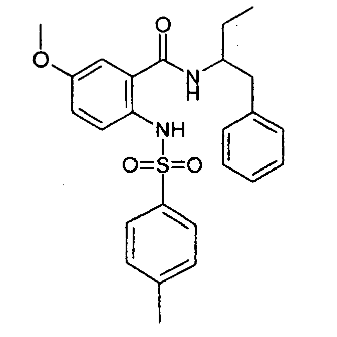 | 453 (M+1) |
| 52 | 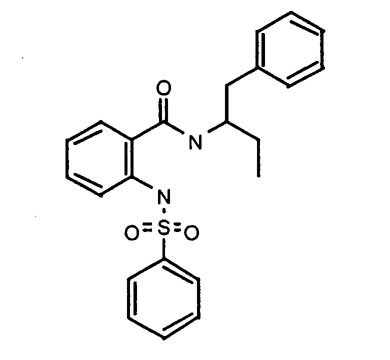 | 409 (M+1) |
| 53 | 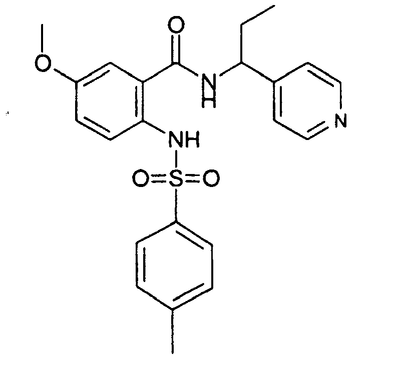 | 440 (M+1) |

### Allgemeine Vorschrift 6: Umsetzung von Sulfonylaminobenzoesäuren mit Aminen

Zu einer Lösung von 0,42 mmol der entsprechenden Sulfonylaminobenzoesäure, 0,44 mmol HOBT und 0,44 mmol EDAC in 5 ml THF werden bei 0°C 0,44 mmol des jeweiligen Amins zugetropft und es wird 4 bis 12 h bei RT gerührt. Die Reaktionsmischung wird mit EE verdünnt und mit verdünnter Salzsäure und Natriumbicarbonatlösung gewaschen. Nach Trocknen über Magnesiumsulfat und Einengen im Vakuum erhält man das entsprechende Amid, das falls erforderlich über präparative HPLC gereinigt wird.

### Beispiel 54: 2-(Butylsulfonylamino)-N-cyclohexyl-5-methyl-benzamid

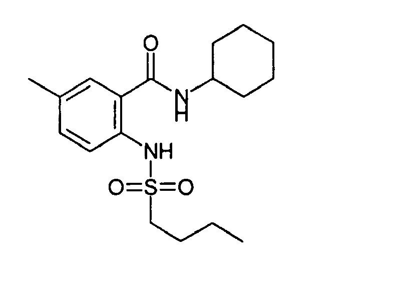

Aus 200 mg 2-Butylsulfonylamino-5-methyl-benzoesäure (Vorstufe 1 b) und Cyclohexylamin wurden gemäß der allgemeinen Vorschrift 6 184 mg der Titelverbindung erhalten. MS (ES+): m/z = 353 (M+1).

Gemäss der allgemeinen Vorschrift 6 wurden unter anderem folgende weitere Beispiele erhalten:

| Beispiel | Struktur | Masse |
|---|---|---|
| 55 | 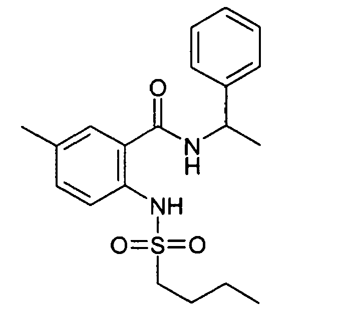 | 375 (M+1) |
| 56 | 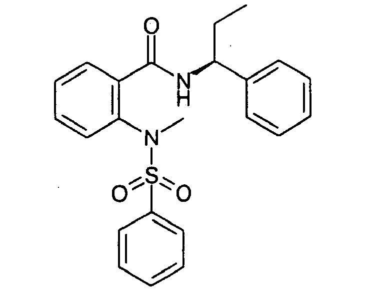 | 409 (M+1) |
| 57 | 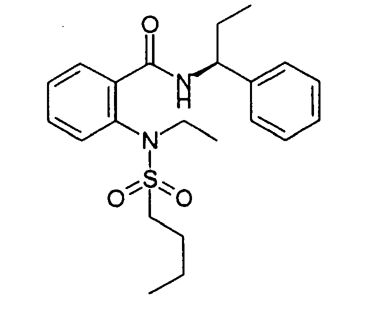 | 403 (M+1) |
| 58 | 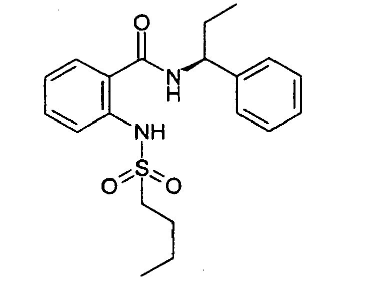 | 375 (M+1) |

### Allgemeine Vorschrift 7: Umsetzung von 2-Aminobenzoesäureamiden mit Sulfonylchloriden

Zu einer Lösung von 0,2 mmol des entsprechenden 2-Aminobenzoesäureamids (Vorstufe 4) und 0,6 mmol Pyridin in 5 ml Methylenchlorid wird eine Lösung von 0,3 mmol des entsprechenden Sulfonylchlorids in 2 ml Methylenchlorid bei 0°C zugetropft, und es wird über Nacht bei RT gerührt. Die organische Phase wird mit Wasser, verdünnter Salzsäure und Natriumbicarbonatlösung gewaschen und das erhaltene Rohprodukt wird, falls erforderlich, über präparative HPLC gereinigt.

Auf diese Weise wurden unter anderem folgende Produkte erhalten:

| Beispiel | Struktur | Masse |
|---|---|---|
| 59 | 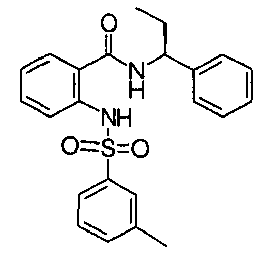 | 409 (M+1) |
| 60 | 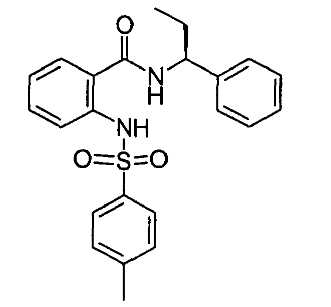 | 409 (M+1) |
| 61 | 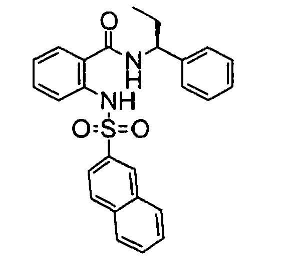 | 445 (M+1) |
| 62 | 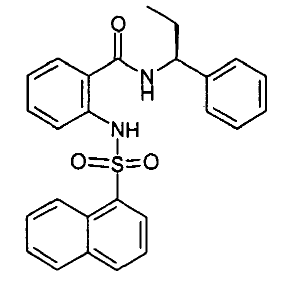 | 445 (M+1) |
| 63 | 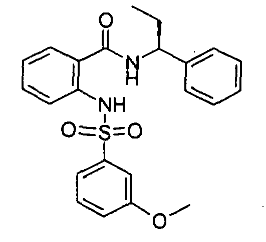 | 425 (M+1) |
| 64 | 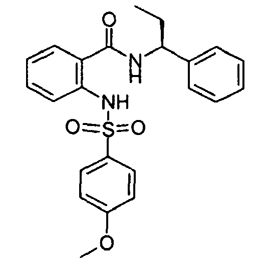 | 425 (M+1) |
| 65 | 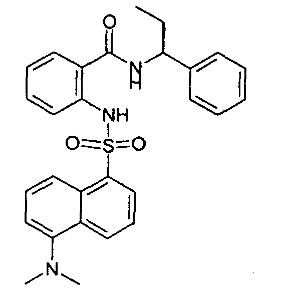 | 488 (M+1) |
| 66 | 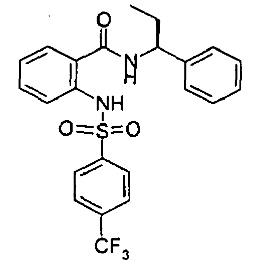 | 463 (M+1) |
| 67 | 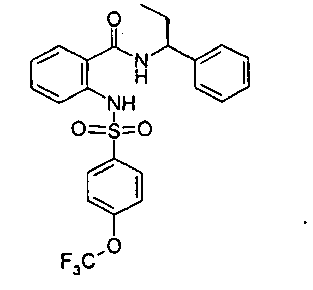 | 479 (M+1) |
| 68 | 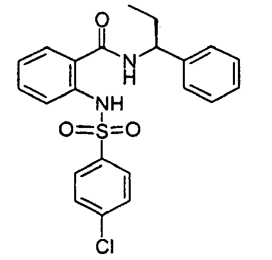 | 429 (M+1) |
| 69 | 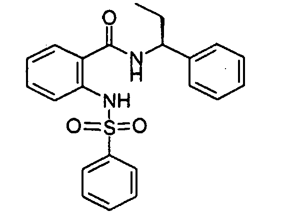 | 395 (M+1) |
| 70 | 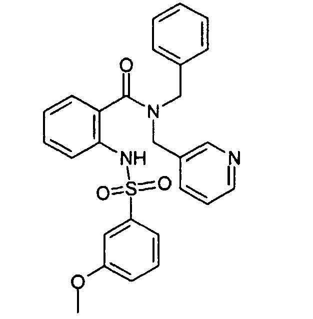 | 488 (M+1) |
| 71 | 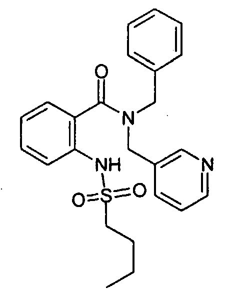 | 438 (M+1) |
| 72 | 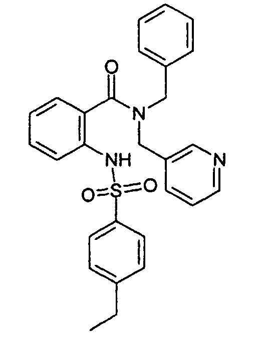 | 486 (M+1) |
| 73 | 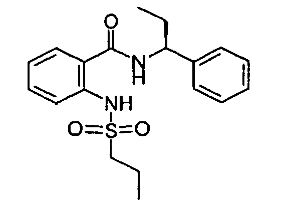 | 361 (M+1) |
| 74 | 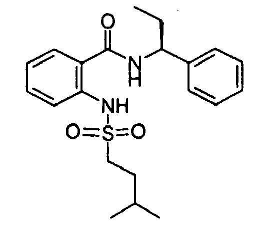 | 389 (M+1) |
| 75 | 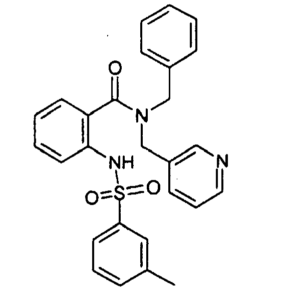 | 472 (M+1) |
| 76 | 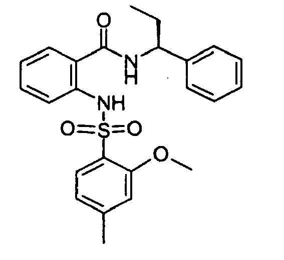 | 439 (M+1) |
| 77 | 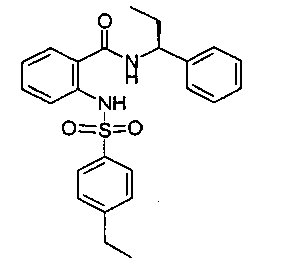 | 423 (M+1) |

Das für Beispiel 74 benötigte 3-Methylbutylsulfonylchlorid wurde hergestellt aus 3-Methylbutylbromid durch Umsetzung mit Ammoniumsulfitlösung unter Rückfluss zur Sulfonsäure gefolgt vom Chlorierung mit Phosphorpentachlorid zum Sulfonylchlorid.

In Analogie zu den obigen Beispielen und durch Anwendung einer oder mehrerer der allgemeinen Vorschriften 1 - 7 wurden weiterhin folgende Verbindungen erhalten:

| Beispiel | Struktur | Masse |
|---|---|---|
| 78 | 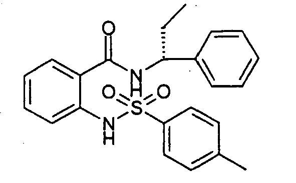 | 409 (M+1) |
| 79 | 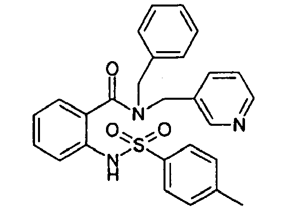 | 472 (M+1) |
| 80 | 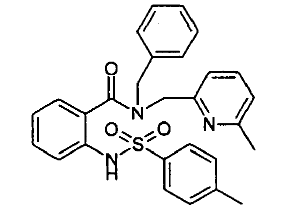 | 485 (M+1) |
| 81 | 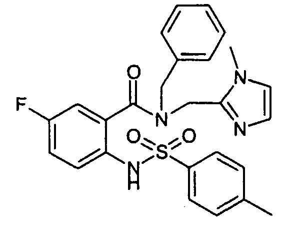 | 493 (M+1) |
| 82 | 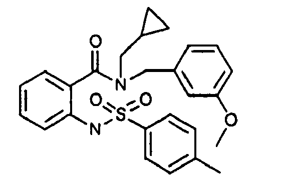 | 465 (M+1) |
| 83 | 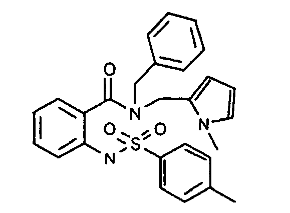 | 474 (M+1) |
| 84 | 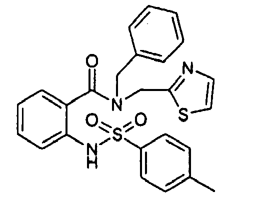 | 478 (M+1) |
| 85 | 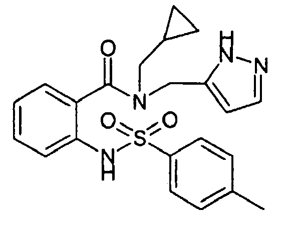 | 425 (M+1) |
| 86 | 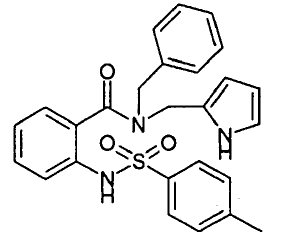 | 460 (M+1) |
| 87 | 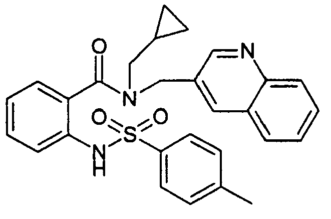 | 486 (M+1) |
| 88 | 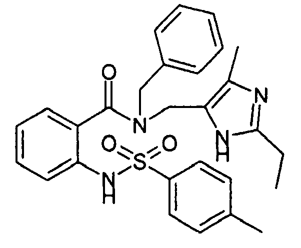 | 503 (M+1) |
| 89 | 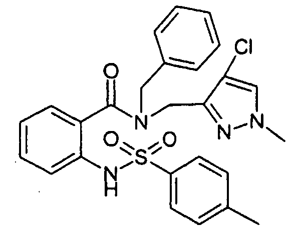 | 509 (M+1) |
| 90 | 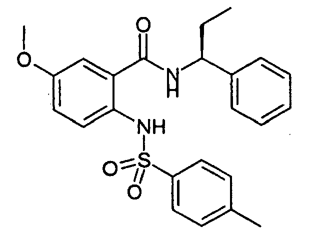 | 439 (M+1) |
| 91 | 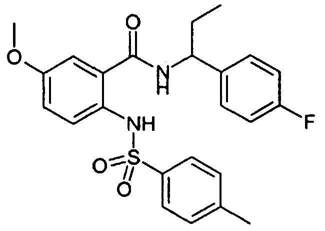 | 457 (M+1) |
| 92 | 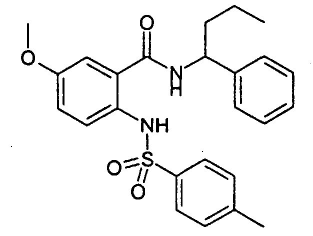 | 453 (M+1) |
| 93 | 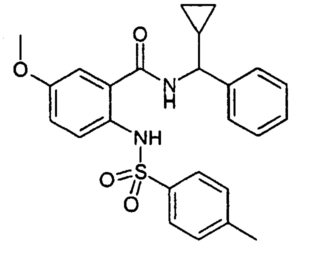 | 451 (M+1) |
| 94 | 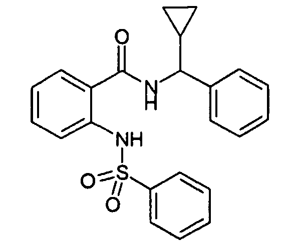 | 407 (M+1) |
| 95 | 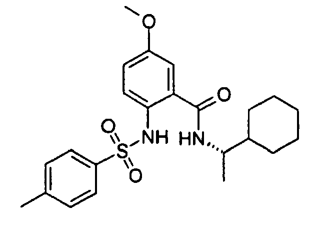 | 431 (M+1) |
| 96 | 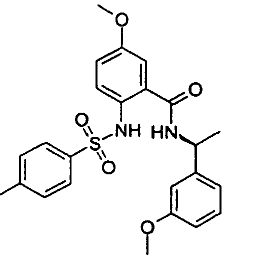 | 455 (M+1) |
| 97 | 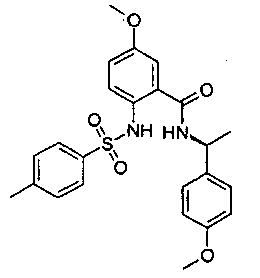 | 455 (M+1) |
| 98 | 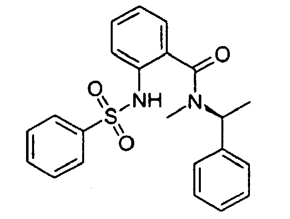 | 395 (M+1) |
| 99 | 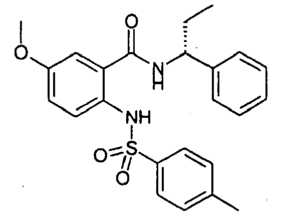 | 439 (M+1) |
| 100 | 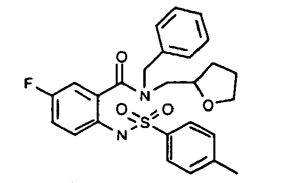 | 483 (M+1) |
| 101 | 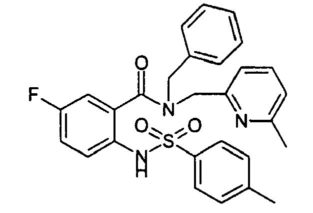 | 504 (M+1) |
| 102 | 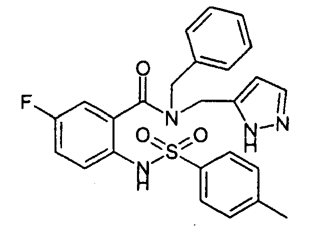 | 479 (M+1) |
| 103 | 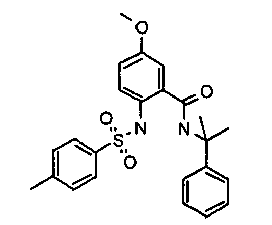 | 439 (M+1) |
| 104 | 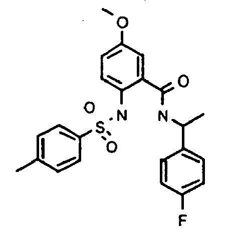 | 443 (M+1) |
| 105 | 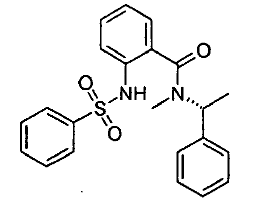 | 395 (M+1) |
| 106 | 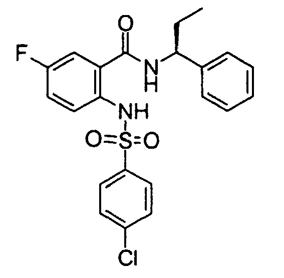 | 447 (M+1) |
| 107 | 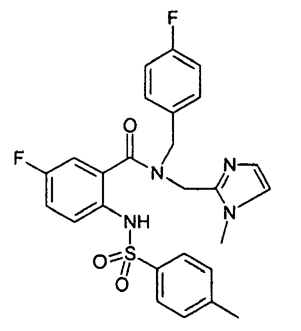 | 511 (M+1) |
| 108 | 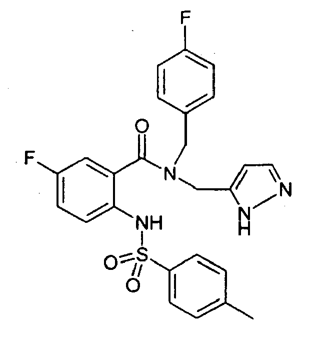 | 497 (M+1) |
| 109 | 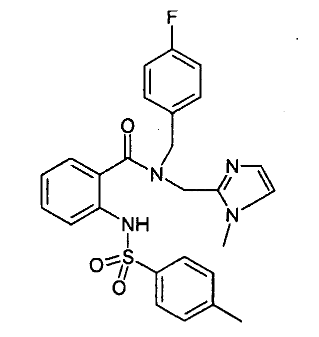 | 493 (M+1) |
| 110 | 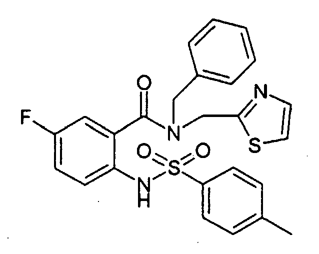 | 496 (M+1) |
| 111 | 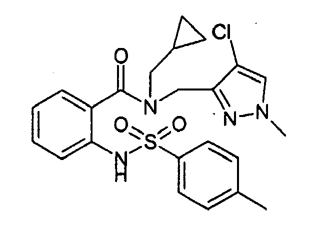 | 473 (M+1) |
| 112 | 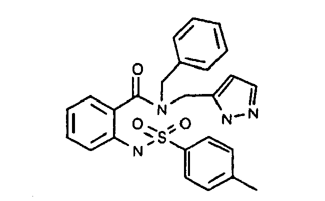 | 461 (M+1) |
| 113 | 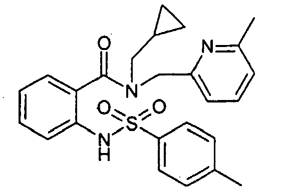 | 450 (M+1) |
| 114 | 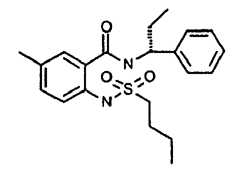 | 389 (M+1) |
| 115 | 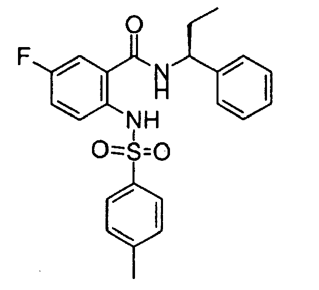 | 427 (M+1) |
| 116 | 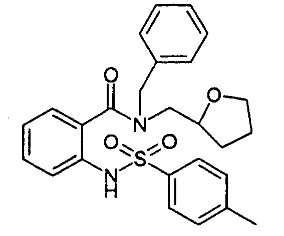 | 465 (M+1) |

Entsprechend der allgemeinen Vorschrift 5 wurden folgende weitere Beispiele hergestellt:

| Beispiel | Struktur | Masse (ES) |
|---|---|---|
| 117 | 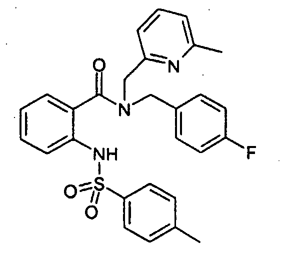 | 504 (M+1) |
| 118 | 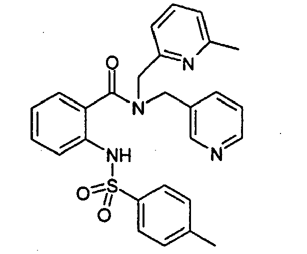 | 487 (M+1) |
| 119 | 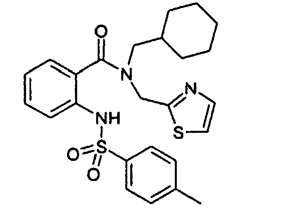 | 484 (M+1) |
| 120 | 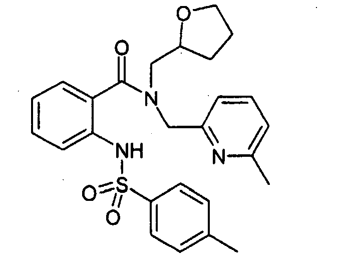 | 480 (M+1) |
| 121 | 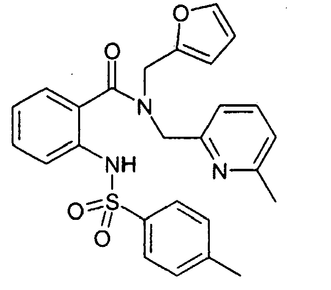 | 476 (M+1) |
| 122 | 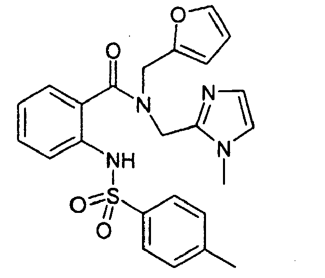 | 465 (M+1) |
| 123 | 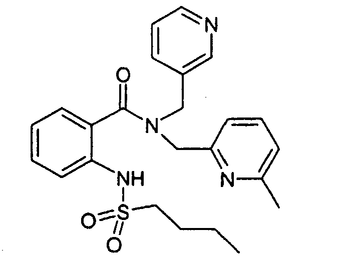 | 453 (M+1) |
| 124 | 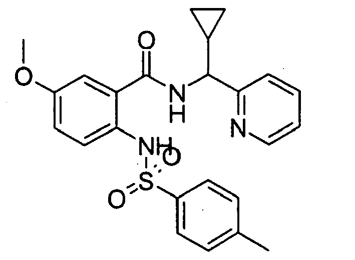 | 452 (M+1) |
| 125 | 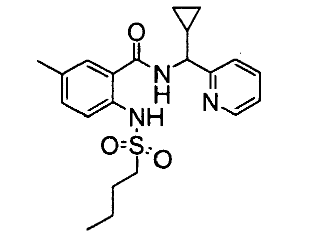 | 402 (M+1) |
| 126 | 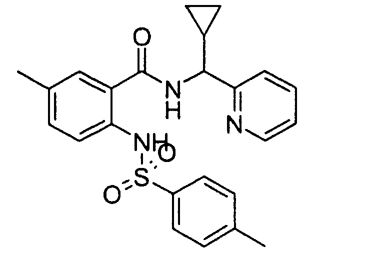 | 436 (M+1) |
| 127 | 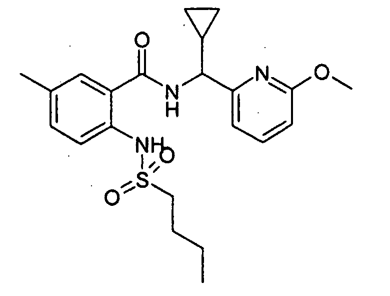 | 432 (M+1) |
| 128 | 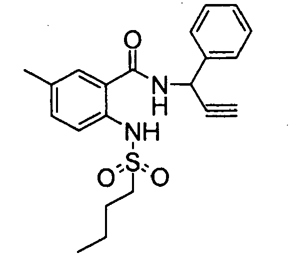 | 385 (M+1) |
| 129 | 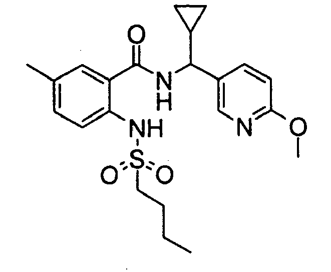 | 432 (M+1) |
| 130 | 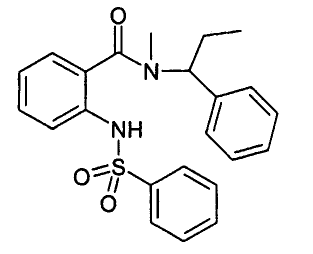 | 409 (M+1) |
| 131 | 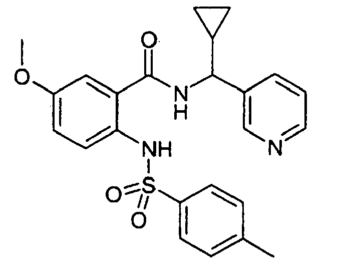 | 452 (M+1) |
| 132 | 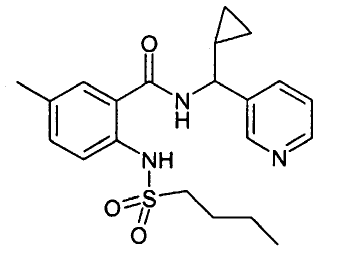 | 402 (M+1) |
| 133 | 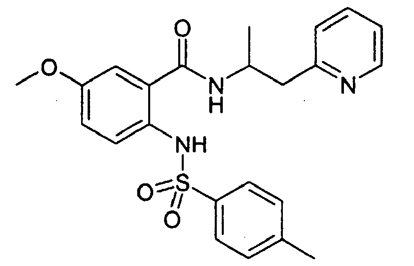 | 440 (M+1) |
| 134 | 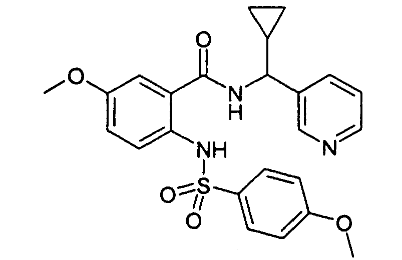 | 468 (M+1) |
| 135 | 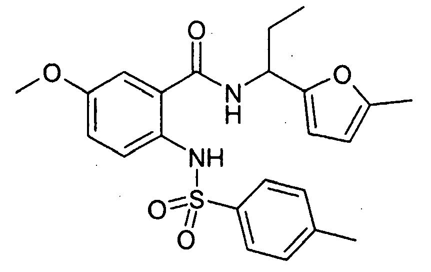 | 443 (M+1) |
| 136 | 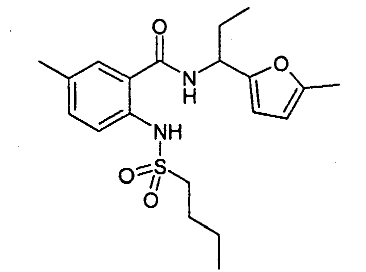 | 393 (M+1) |

Gemäß der allgemeinen Vorschrift 6 wurden folgende weitere Beispiele hergestellt:

| Beispiel | Struktur | Masse (ES) |
|---|---|---|
| 137 | 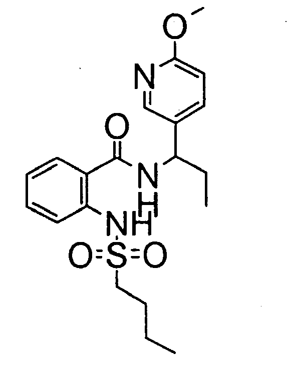 | 406 (M+1) |
| 138 | 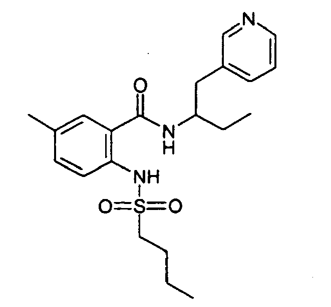 | 404 (M+1) |
| 139 | 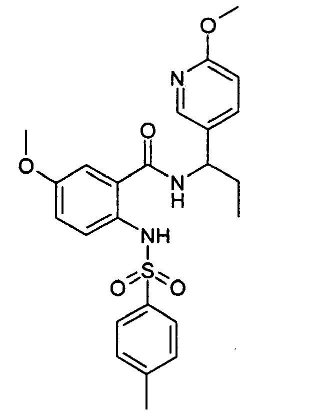 | 470 (M+1) |
| 140 | 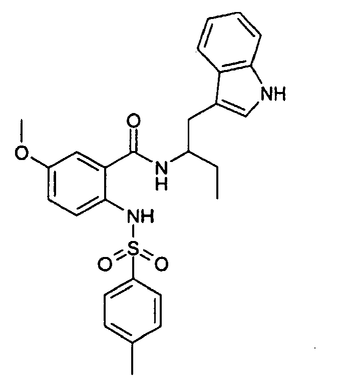 | 492 (M+1) |
| 141 | 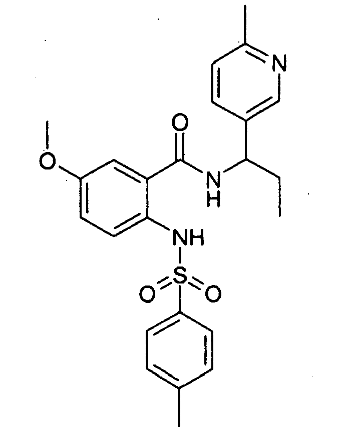 | 454 (M+1) |
| 142 | 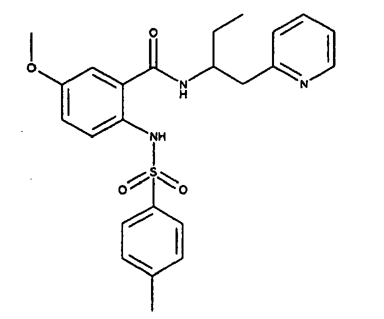 | 454 (M+1) |
| 143 | 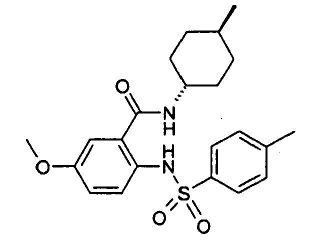 | 417 (M+1) |
| 144 | 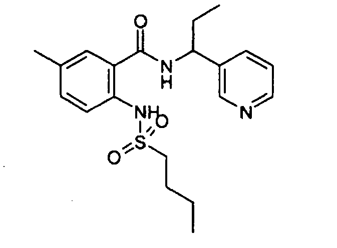 | 390 (M+1) |
| 145 | 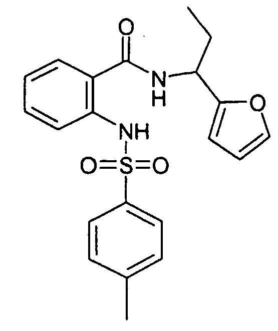 | 399 (M+1) |

Gemäß der allgemeinen Vorschrift 7 wurden folgende weitere Beispiele hergestellt:

| Beispiel | Struktur | Masse (ES) |
|---|---|---|
| 146 | 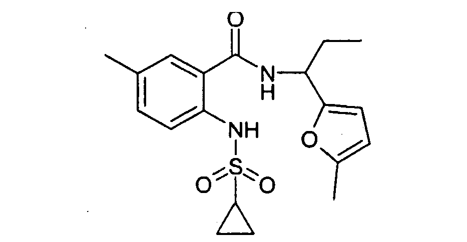 | 377 (M+1) |
| 147 | 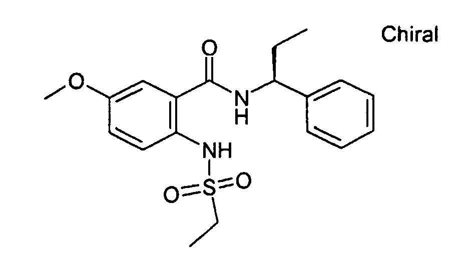 | 377 (M+1) |
| 148 | 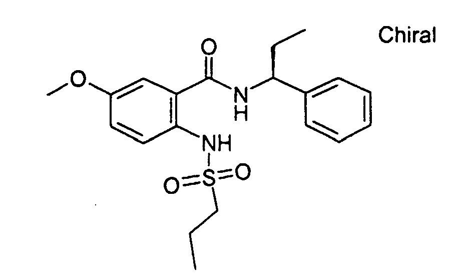 | 391 (M+1) |
| 149 | 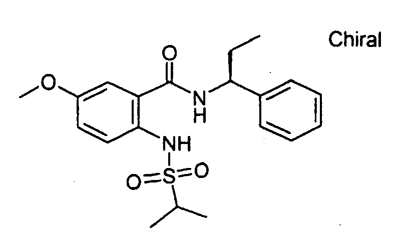 | 391 (M+1) |
| 150 | 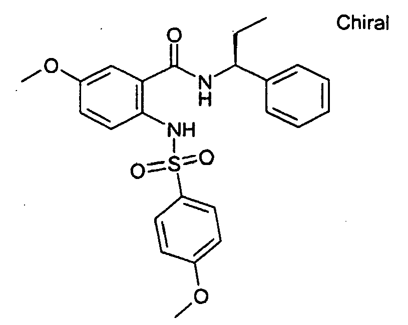 | 455 (M+1) |
| 151 | 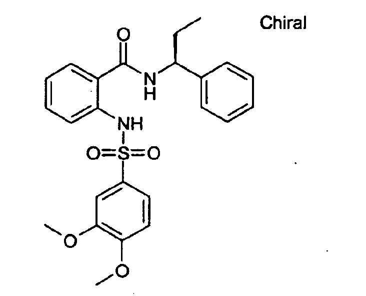 | 455 (M+1) |

Beispiel 152: 5-Hydroxy-N-(1-phenyl-propyl)-2-(toluol-4-sulfonylamino)-benzamid

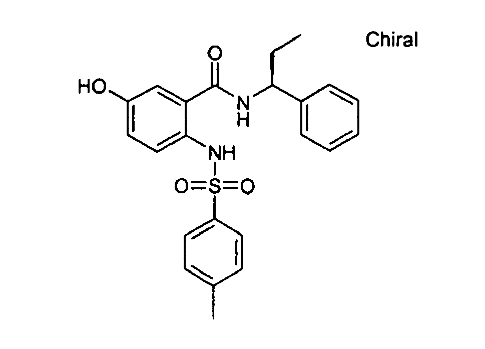

Die Verbindung wurde erhalten aus der Verbindung des Beispiels 90 durch Spaltung des Methylethers mit Bortribromid.

### Pharmakologische Untersuchungen

Kv1.5-Kanäle aus dem Menschen wurden in Xenopus Oozyten exprimiert. Hierfür wurden zuerst Oozyten aus Xenopus laevis isoliert und defollikuliert. Anschließend wurde in diese Oozyten in vitro synthetisierte Kv1.5 kodierende RNA injiziert. Nach 1 - 7 Tagen Kv1.5-Proteinexpression wurden an den Oozyten mit der Zwei-Mikroelektroden Voltage-Clamp Technik Kv1.5-Ströme gemessen. Die Kv1.5-Kanäle wurden hierbei in der Regel mit 500 ms dauernden Spannungssprüngen auf 0 mV und 40 mV aktiviert. Das Bad wurde mit einer Lösung der nachfolgenden Zusammensetzung durchspült: NaCl 96 mM, KCI 2 mM, CaCl₂ 1,8 mM, MgCl₂ 1 mM, HEPES 5 mM (titriert mit NaOH auf pH 7,4). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADlnstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des Kv1.5-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

Auf diese Weise wurden für die nachfolgend aufgeführten Verbindungen folgende IC₅₀-Werte bestimmt:

| Beispiel Nr. | IC₅₀ [µM] |
|---|---|
| 1 | 5,5 |
| 2 | 8,2 |
| 3 | 2,8 |
| 4 | 4,1 |
| 5 | 4,5 |
| 6 | 8,2 |
| 7 | 5,1 |
| 8 | 0,9 |
| 9 | 4,9 |
| 10 | 2,5 |
| 11 | 8,0 |
| 12 | 5,4 |
| 13 | 10,0 |
| 14 | 8,5 |
| 15 | 7,8 |
| 16 | 6,4 |
| 17 | 7,5 |
| 18 | 6,7 |
| 19 | 4,2 |
| 20 | 7,7 |
| 21 | 5,2 |
| 22 | 4,8 |
| 23 | 5,0 |
| 24 | 3,0 |
| 25 | 3,4 |
| 26 | 0,6 |
| 27 | 5,6 |
| 28 | 3,2 |
| 29 | 5,1 |
| 30 | 0,7 |
| 31 | 2,6 |
| 32 | 8,5 |
| 33 | 6,5 |
| 34 | 3,1 |
| 35 | 2,0 |
| 36 | 2,0 |
| 37 | 1,9 |
| 38 | 1,6 |
| 39 | > 10 |
| 40 | 0,8 |
| 41 | 1,9 |
| 42 | 5,0 |
| 43 | 5,4 |
| 44 | 2,9 |
| 45 | 2,5 |
| 46 | 1,2 |
| 47 | 4,7 |
| 48 | > 10 |
| 49 | 6,3 |
| 50 | 0,7 |
| 51 | 3,6 |
| 52 | 3,0 |
| 53 | 2,5 |
| 54 | 4,0 |
| 55 | 4,6 |
| 56 | 10,0 |
| 57 | 5,0 |
| 58 | >10 |
| 59 | 1,7 |
| 60 | 0,7 |
| 61 | 5,6 |
| 62 | 3,5 |
| 63 | 1,7 |
| 64 | 0,6 |
| 65 | 4,8 |
| 66 | 4,0 |
| 67 | 5,8 |
| 68 | 2,9 |
| 69 | 1,3 |
| 70 | 3,8 |
| 71 | 6,9 |
| 72 | 5,0 |
| 73 | 3,1 |
| 74 | 1,7 |
| 75 | 2,9 |
| 76 | 4,7 |
| 77 | 2,6 |
| 78 | 2,7 |
| 79 | 0,7 |
| 80 | 1,2 |
| 81 | 0,4 |
| 82 | 3,2 |
| 83 | 1,9 |
| 84 | 1,0 |
| 85 | 5,2 |
| 86 | 1,8 |
| 87 | 6,0 |
| 88 | 3,9 |
| 89 | 3,2 |
| 90 | 0,5 |
| 91 | 0,8 |
| 92 | 0,9 |
| 93 | 1,1 |
| 94 | 1,0 |
| 95 | 0,7 |
| 96 | 1,0 |
| 97 | 0,9 |
| 98 | 2,0 |
| 99 | 0,9 |
| 100 | 1,6 |
| 101 | 0,9 |
| 102 | 0,7 |
| 103 | 1,4 |
| 104 | 1,0 |
| 105 | 2,6 |
| 106 | 3,8 |
| 107 | 0,9 |
| 108 | 1,2 |
| 109 | 1,1 |
| 110 | 1,0 |
| 111 | 1,7 |
| 112 | 0,5 |
| 113 | 2,6 |
| 114 | 1,4 |
| 115 | 2,4 |
| 116 | 1,1 |
| 117 | 2,4 |
| 118 | 2,2 |
| 119 | 2,7 |
| 120 | 1,4 |
| 121 | 0,6 |
| 122 | 1,2 |
| 123 | 2,9 |
| 124 | 1,0 |
| 125 | 1,8 |
| 126 | 0,6 |
| 127 | 3,3 |
| 128 | 2,2 |
| 129 | 1,3 |
| 130 | 0,7 |
| 131 | 0,7 |
| 132 | 1,0 |
| 133 | 1,4 |
| 134 | 0,9 |
| 135 | 1,2 |
| 136 | 0,9 |
| 137 | 2,4 |
| 138 | 1,8 |
| 139 | 1,0 |
| 140 | 1,4 |
| 141 | 1,1 |
| 142 | 0,7 |
| 143 | 3,6 |
| 144 | 2,8 |
| 145 | 2,6 |
| 146 | 2,5 |
| 147 | 3,8 |
| 148 | 2,1 |
| 149 | 2,2 |
| 150 | 0,3 |
| 151 | 1,5 |
| 152 | 2,6 |

## Patentansprüche

1. Verbindungen der Formel I, 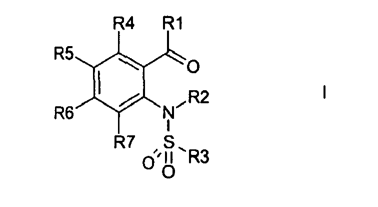 worin bedeuten: 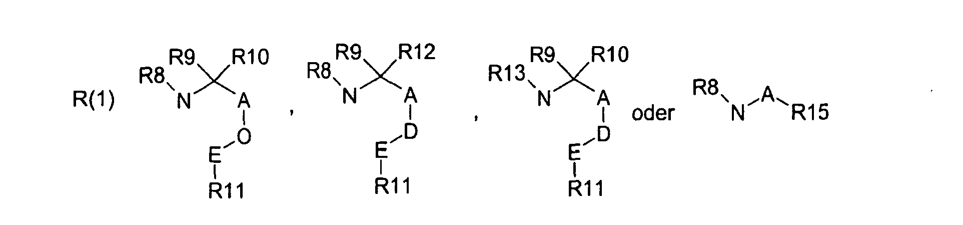
A -CₙH₂ₙ-;
n 0, 1, 2, 3, 4 oder 5;
O Sauerstoff;
D eine Bindung oder Sauerstoff;
E -CₘH₂ₘ-;
m 0, 1, 2, 3, 4 oder 5;
R(8) Wasserstoff , Alkyl mit 1, 2, 3 oder 4 C-Atomen oder CₚH₂ₚ-R(14);
p 0, 1, 2, 3, 4 oder 5;
R(14) Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3, 4, 5 oder 6 C-Atomen;
R(10) Wasserstoff, Alkyl mit 1, 2, 3 oder 4 C-Atomen, Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Cycloalkyl mit 3, 4, 5 oder 6 C-Atomen, Phenyl, Naphthyl, Thienyl, Furyl, Pyridyl, Pyrazinyl, Pyridazinyl oder Pyrimidyl, wobei Phenyl, Naphthyl, Thienyl, Furyl, Pyridyl, Pyrazinyl, Pyridazinyl und Pyrimidyl unsubstituiert sind oder substituiert mit 1, 2 oder 3 Substituenten ausgewählt aus der Gruppe bestehend aus F, Cl, Br, I, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2, 3 oder 4 C-Atomen, Cycloalkyl mit 3, 4, 5 oder 6 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14');
p 0, 1, 2, 3, 4 oder 5;
R(14') Cycloalkyl mit 3, 4, 5 oder 6 C-Atomen, Tetrahydrofuranyl, Tetrahydropyranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Cycloalkyl mit 3, 4, 5, 6, 7 oder 8 C-Atomen;
R(2) Wasserstoff oder Alkyl mit 1, 2, 3 oder 4 C-Atomen; .
R(3) Alkyl mit 3, 4, 5, 6 oder 7 C-Atomen, Cycloalkyl mit 3, 4, 5 oder 6 C-Atomen, Phenyl oder Naphthyl, wobei Phenyl oder Naphthyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, Br, I, CF₃, OCF₃, OCHF₂, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
wobei die Cycloalkylreste verzweigt sein können;
sowie ihre pharmazeutisch akzeptablen Salze.

2. Verbindungen der Formel I nach Anspruch 1, worin bedeuten: 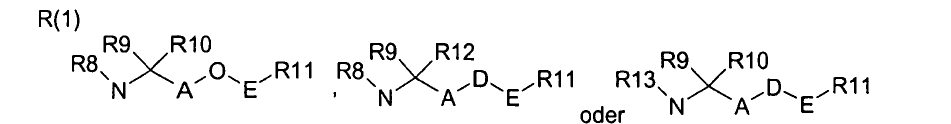
A -CₙH₂ₙ-;
n 0, 1, 2, 3, 4 oder 5;
O Sauerstoff;
D eine Bindung oder Sauerstoff;
E -CₘH₂ₘ-;
m 0, 1, 2, 3, 4 oder 5;
R(8) Wasserstoff , Alkyl mit 1, 2, 3 oder 4 C-Atomen oder CₚH₂ₚ-R(14);
p 0, 1, 2, 3, 4 oder 5;
R(14) Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3, 4, 5 oder 6 C-Atomen;
R(10) Wasserstoff, Alkyl mit 1, 2, 3 oder 4 C-Atomen, Cycloalkyl mit 3, 4, 5 oder 6 C-Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Cycloalkyl mit 3, 4, 5 oder 6 C-Atomen, Phenyl oder Pyridyl, wobei Phenyl und Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2, 3 oder 4 C-Atomen, Cycloalkyl mit 3, 4, 5 oder 6 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14');
p 0, 1, 2, 3, 4 oder 5;
R(14') Cycloalkyl mit 3, 4, 5 oder 6 C-Atomen, Tetrahydrofuranyl, Tetrahydropyranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2, 3 oder 4 C-Atomen;
R(3) Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, Br, I, CF₃, OCF₃, OCHF₂, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

3. Verbindungen der Formel I nach Ansprüchen 1 oder 2, worin bedeuten: 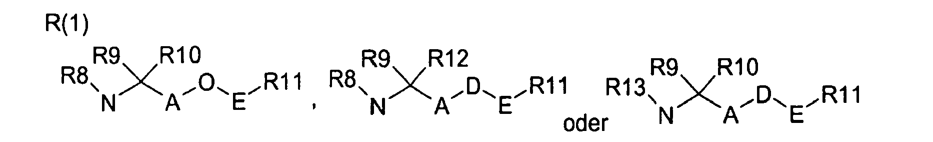
A -CₙH₂ₙ-;
n 0, 1, 2 oder 3;
O Sauerstoff;
D eine Bindung oder Sauerstoff;
E -CₘH₂ₘ-;
m 0, 1, 2 oder 3;
R(8) Wasserstoff oder Alkyl mit 1, 2, 3 oder 4 C-Atomen;
R(9) Wasserstoff oder Alkyl mit 1, 2, 3, 4, 5 oder 6 C-Atomen;
R(10) Wasserstoff, Alkyl mit 1, 2, 3 oder 4 C-Atomen oder Cycloalkyl mit 3, 4 oder 5 C-Atomen,
R(11) Cycloalkyl mit 3, 4, 5 oder 6 C-Atomen, Phenyl oder Pyridyl, wobei Phenyl und Pyridyl unsubstituiert sind oder substituiert mit 1, 2 oder 3 Substituenten ausgewählt aus der Gruppe bestehend aus F, Cl, Br, CF₃, OCF₃, NO₂, CN, COMe, OH, Alkyl mit 1, 2, 3 oder 4 C-Atomen, Alkoxy mit 1, 2, 3 oder 4 C- Atomen, Dimethylamino, Sulfamoyl, Methylsulfonyl und Methylsulfonylamino;
R(12) Alkyl mit 1, 2, 3 oder 4 C-Atomen, Cycloalkyl mit 3, 4 oder 5 C- Atomen, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14');
p 0, 1, 2, 3, 4 oder 5;
R(14') Cycloalkyl mit 3, 4, 5 oder 6 C-Atomen, Tetrahydrofuranyl, Tetrahydropyranyl, Aryl oder Heteroaryl, wobei Aryl und Heteroaryl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1 oder 2 C-Atomen;
R(3) Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, Br, CF₃, OCF₃, NO₂, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, Br, CF₃, OCF₃, OCHF₂, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

4. Verbindungen der Formel I nach Ansprüchen 1 - 3, worin bedeuten: R1 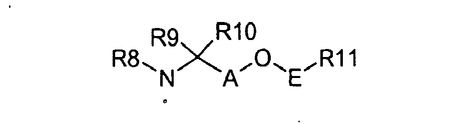
A -CₙH₂ₙ-;
n = 0, 1 oder 2;
O Sauerstoff;
E -CₘH₂ₘ-;
m 0 oder 1;
R(8) Wasserstoff oder Alkyl mit 1, 2, 3 oder 4 C-Atomen;
R(9) Wasserstoff oder Alkyl mit 1, 2, 3 oder 4 C-Atomen;
R(10) Wasserstoff oder Alkyl mit 1 oder 2 C-Atomen
R(11) Phenyl, wobei Phenyl unsubstituiert ist oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
R(2) Wasserstoff;
R(3) Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

5. Verbindungen der Formel I nach Ansprüche 1 - 3, worin bedeuten: 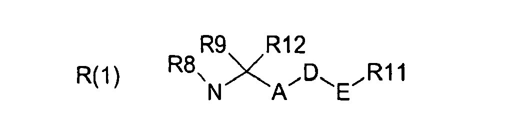
A -CₙH₂ₙ-;
n 0, 1 oder 2;
D eine Bindung oder Sauerstoff;
E -CₘH₂ₘ-;
m 0 oder 1;
R(8) Wasserstoff oder Alkyl mit 1, 2, 3 oder 4 C-Atomen;
R(9) Wasserstoff oder Alkyl mit 1, 2, 3 oder 4 C-Atomen;
R(11) Phenyl oder Pyridyl, wobei Phenyl und Pyridyl unsubstituiert sind oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino; R(12) Alkyl mit 1, 2, 3 oder 4 C-Atomen oder Cyclopropyl;
R(2) Wasserstoff;
R(3) Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

6. Verbindungen der Formel I nach Ansprüchen 1 - 3, worin bedeuten: 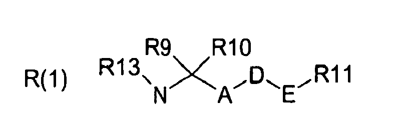
A -CₙH₂ₙ-;
n 0, 1 oder 2;
D eine Bindung oder Sauerstoff;
E -CₘH₂ₘ-;
m = 0 oder 1;
R(9) Wasserstoff oder Alkyl mit 1 oder 2 C-Atomen;
R(10) Wasserstoff oder Alkyl mit 1 oder 2 C-Atomen;
R(11) Cycloalkyl mit 3, 4, 5 oder 6 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, CN, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14');
p 0, 1, 2, 3 oder 4;
R(14') Aryl oder Heteroaryl, wobei Aryl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff;
R(3) Alkyl mit 3, 4 oder 5 C-Atomen, Phenyl, wobei Phenyl unsubstituiert ist oder substituiert mit 1, 2 oder 3 Substituenten ausgewählt aus der Gruppe bestehend aus F, Cl, CF₃, OCF₃, COOMe, CONH₂, COMe, OH, Alkyl mit 1, 2, 3 oder 4 C- Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch akzeptablen Salze.

7. Verbindung der Formel I nach einem oder mehreren der Ansprüche 1 bis 3 und 5, die ist

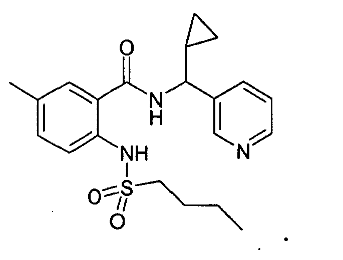

8. Verbindung der Formel I nach einem oder mehreren der Ansprüche 1 bis 3 und 5, die ist

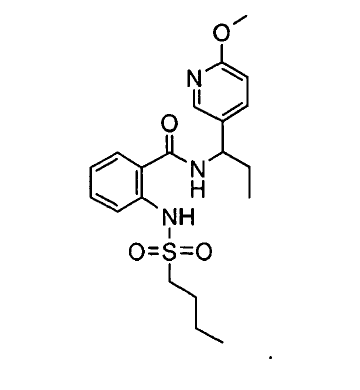

9. Verbindungen der Formel I nach einem oder mehreren der Ansprüche 1 bis 8 und ihre physiologisch verträglichen Salze zur Anwendung als Arzneimittel.

10. Pharmazeutische Zubereitung, enthaltend eine wirksame Menge mindestens einer Verbindung der Formel I nach einem oder mehreren der Ansprüche 1 bis 8 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

11. Verwendung einer Verbindung der Formel I nach einem oder mehreren der Ansprüche 1 bis 8 und/oder eines physiologisch verträglichen Salzes davon zur Herstellung eines Medikaments mit K⁺-Kanal-blockierender Wirkung zur Therapie und Prophylaxe von K⁺-Kanal mediierten, Krankheiten.

12. Verwendung einer Verbindung der Formel I nach einem oder mehreren der Ansprüche 1 bis 8 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

13. Verwendung einer Verbindung der Formel I nach einem oder mehreren der Ansprüche 1 bis 8 und/oder eines physiologisch verträglichen Salzes davon zur Herstellung eines Medikaments zur Therapie oder Prophylaxe von Reentry-Arrhythmien.

14. Verwendung einer Verbindung der Formel I nach einem oder mehreren der Ansprüche 1 bis 8 und/oder eines physiologisch verträglichen Salzes davon zur Herstellung eines Medikaments zur Therapie oder Prophylaxe von supraventrikulären Arrhythmien.

15. Verwendung einer Verbindung der Formel I nach einem oder mehreren der Ansprüche 1 bis 8 und/oder eines physiologisch verträglichen Salzes davon zur Herstellung eines Medikaments zur Therapie oder Prophylaxe von atrialer Fibrillation oder atrialem Flattern.

16. Pharmazeutische Zubereitung, enthaltend eine wirksame Menge mindestens einer Verbindung der Formel I nach einem oder mehreren der Ansprüche 1 bis 8 und/oder eines physiologisch verträglichen Salzes davon sowie eines Beta-blockers als Wirkstoffe, zusammen mit pharmazeutisch annehmbaren Träger- und Zusatzstoffen.

17. Pharmazeutische Zubereitung, enthaltend eine wirksame Menge mindestens einer Verbindung der Formel I nach einem oder mehreren der Ansprüche 1 bis 8 und/oder eines physiologisch verträglichen Salzes davon sowie eines IKs-Kanalblockers als Wirkstoffe, zusammen mit pharmazeutisch annehmbaren Träger- und Zusatzstoffen.

## Claims

1. Compounds of the formula I, 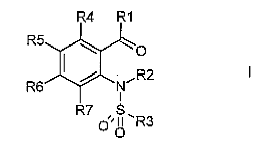 in which: 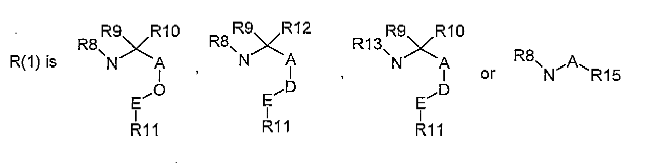
A is -CₙH₂ₙ-;
n is 0, 1, 2, 3, 4 or 5;
O is oxygen;
D is a bond or oxygen;
E is -CₘH₂ₘ-;
m is 0, 1, 2, 3, 4 or 5;
R(8) is hydrogen, alkyl having 1, 2, 3 or 4 carbon atoms or CₚH₂ₚ-R(14);
p is 0, 1, 2, 3, 4 or 5;
is cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, CCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(9) is hydrogen or alkyl having 1, 2, 3, 4, 5 or 6 carbon atoms;
R(10) is hydrogen, alkyl having 1, 2, 3 or 4 carbon atoms, cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(11) is cycloalkyl having 3, 4, 5 or 6 carbon atoms, phenyl, naphthyl, thienyl, furyl, pyridyl, pyrazinyl, pyridazinyl or pyrimidyl, where phenyl, naphthyl, thienyl, furyl, pyridyl, pyrazinyl, pyridazinyl and pyrimidyl are unsubstituted or substituted by 1, 2 or 3 substituents selected from the group consisting of F, Cl, Br, I, CF₃, OCF₃, NO₂, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(12) is alkyl having 1, 2, 3 or 4 carbon atoms, cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, Alkoxy having 1, 2, 3 or 4 carbon atoms, dimethylamino, sulfamoyl, Methylsulfonyl and methylsulfonylamino;
R(13) is CₚH₂ₚ-R(14');
p is 0, 1, 2, 3, 4 or 5;
R(14') is cycloalkyl having 3, 4, 5 or 6 carbon atoms, tetrahydrofuranyl, tetrahydropyranyl, aryl or heteroaryl, where aryl and heteroaryl are unsubsituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(15) is cycloalkyl having 3, 4, 5, 6, 7 or 8 carbon atoms;
R(2) is hydrogen or alkyl having 1, 2, 3 or 4 carbon atoms;
R(3) is alkyl having 3, 4, 5, 6 or 7 carbon atoms, cycloalkyl having 3, 4, 5 or 6 carbon atoms, phenyl or naphthyl, where phenyl or naphthyl are unsubstituted or substituted by 1, 2 or 3 substituents selected from the group consisting of F, Cl, Br, I, CF₃, CCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, Br, I, CF₃, CCF₃, OCHF₂, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
where the cycloalkyl radicals can be branched;
and the pharmaceutically acceptable salts thereof.

2. Compounds of the formula I according to Claim 1, in which: 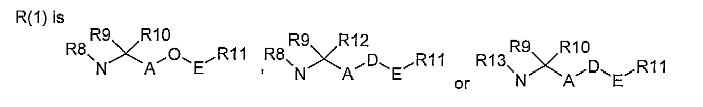
A is -CₙH₂ₙ-;
n is 0, 1, 2, 3, 4 or 5;
O is oxygen;
D is a bond or oxygen;
E is -CₘH₂ₘ-;
m is 0, 1, 2, 3, 4 or 5;
R(8) is hydrogen, alkyl having 1, 2, 3 or 4 carbon atoms or CₚH₂ₚ-R(14); p is 0, 1, 2, 3, 4 or 5; R(14) is cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethyl- amino, sulfamoyl, methylsulfonyl and methylsulfonylamino;
R(9) is hydrogen or alkyl having 1, 2, 3, 4, 5 or 6 carbon atoms;
R(10) is hydrogen, alkyl having 1, 2, 3 or 4 carbon atoms, cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, Methylsulfonyl and methylsulfonylamino;
R(11) is cycloalkyl having 3, 4, 5 or 6 carbon atoms, phenyl or pyridyl, where phenyl and pyridyl are unsubstituted or substituted by 1, 2 or 3 substituents selected from the group consisting of F, Cl, Br, I, CF₃, OCF₃, NO₂, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(12) is alkyl having 1, 2, 3 or 4 carbon atoms, cycloalkyl having 3, 4, 5 or 6 carbon atoms, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is CₚH₂ₚ-R(14');
p is 0, 1, 2, 3, 4 or 5;
R(14')is cycloalkyl having 3, 4, 5 or 6 carbon atoms, tetrahydrofuranyl, tetrahydropyranyl, aryl or heteroaryl, where aryl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1, 2, 3 or 4 carbon atoms;
R(3) is alkyl having 3, 4 or 5 carbon atoms, phenyl, where phenyl is unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, Br, I, CF₃, OCF₃, OCHF₂, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

3. Compounds of the formula I according to Claims 1 or 2, in which: 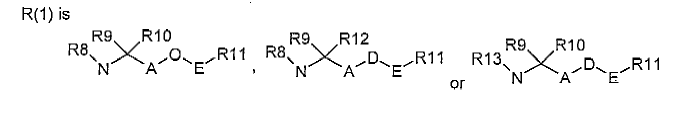
A is --CₙH₂ₙ-;
n is 0, 1, 2 or 3;
O is oxygen;
D is a bond or oxygen;
E is -CₘH₂ₘ-;
m is 0, 1, 2 or 3;
R(8) is hydrogen or alkyl having 1, 2, 3 or 4 carbon atoms;
R(9) is hydrogen or alkyl having 1, 2, 3, 4, 5 or 6 carbon atoms;
R(10) is hydrogen, alkyl having 1, 2, 3 or 4 carbon atoms or cycloalkyl having 3, 4 or 5 carbon atoms,
R(11) is cycloalkyl having 3, 4, 5 or 6 carbon atoms, phenyl or pyridyl, where phenyl and pyridyl are unsubstituted or substituted by 1, 2 or 3 substituents selected from the group consisting of F, Cl, Br, CF₃, OCF₃, NO₂, CN, COMe, OH, alkyl having 1, 2, 3 or 4 carbon atoms, alkoxy having 1, 2, 3 or 4 carbon atoms, dimethylamino, sulfamoyl, Methylsulfonyl and methylsulfonylamino;
is alkyl having 1, 2, 3 or 4 carbon atoms, cycloalkyl having 3, 4 or 5 carbon atoms, aryl or heteroaryl, where aryl and heteroaryl are unsubstituted or substituted by 1, 2 or 3 substituents selected from the group consisting of F, Cl, Br, CF₃, OCF₃, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is CₚH₂ₚ-R(14');
p is 0, 1, 2, 3, 4 or 5;
R(14') is cycloalkyl having 3, 4, 5 or 6 carbon atoms, tetrahydrofuranyl, tetrahydropyranyl, aryl or heteroaryl, where aryl and heteroaryl are unsubstituted or substituted by 1, 2 or 3 substituents selected from the group consisting of F, Cl, Br, CF₃, OCF₃, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1 or 2 carbon atoms;
R(3) is alkyl having 3, 4 or 5 carbon atoms, phenyl, where phenyl is unsubstituted or substituted by 1, 2 or 3 substituents selected from the group consisting of F, Cl, Br, CF₃, OCF₃, NO₂, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, Br, CF₃, OCF₃, OCHF₂, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

4. Compounds of the formula I according to Claims 1-3, in which: 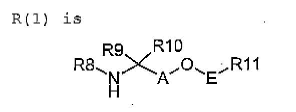
A is -CₙH₂ₙ-;
n is 0, 1 or 2;
O is oxygen;
E is -CₘH₂ₘ-;
m is 0 or 1;
R(8) is hydrogen or alkyl having 1, 2, 3 or 4 carbon atoms;
R(9) is hydrogen or alkyl having 1, 2, 3 or 4 carbon atoms;
R(10) is hydrogen or alkyl having 1 or 2 carbon atoms
R(11) is phenyl,
where phenyl is unsubstituted or substituted by 1, 2 or 3 substituents selected from the group consisting of F, Cl, CF₃, OCF₃, CN, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen;
R(3) is alkyl having 3, 4 or 5 carbon atoms, phenyl, where phenyl is unsubstituted or substituted by 1, 2 or 3 substituents selected from the group consisting of F, Cl, CF₃, OCF₃, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

5. Compounds of the formula I according to Claims 1-3, in which: 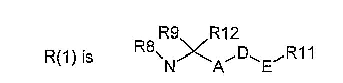
A is -CₙH₂ₙ-;
n is 0, 1 or 2;
D is a bond or oxygen;
E is -CₘH₂ₘ-;
m is 0 or 1;
R(8) is hydrogen or alkyl having 1, 2, 3 or 4 carbon atoms;
R(9) is hydrogen or alkyl having 1, 2, 3 or 4 carbon atoms;
R(11) is phenyl or pyridyl, where phenyl and pyridyl are unsubstituted or substituted by 1, 2 or 3 substituents selected from the group consisting of F, Cl, CF₃, OCF₃, CN, COMe, OH, alkyl having 1, 2, 3 or 4 carbon atoms, alkoxy having 1, 2, 3 or 4 4 carbon atoms, dimethylamino, sulfamoyl, methylsulfonyl and methylsulfonylamino;
R(12) is alkyl having 1, 2, 3 or 4 carbon atoms or cyclopropyl;
R(2) is hydrogen;
R(3) is alkyl having 3, 4 or 5 carbon atoms, phenyl, where phenyl is unsubstituted or substituted by 1, 2 or 3 substituents selected from the group consisting of F, Cl, CF₃, OCF₃, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCE₃, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

6. Compounds of the formula I according to Claims 1-3, in which: 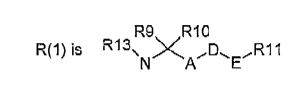
A is -CₙH₂ₙ-;
n is 0, 1 or 2;
D is a bond or oxygen;
E is -CₘH₂ₘ-;
m is 0 or 1;
R(9) is hydrogen or alkyl having 1 or 2 carbon atoms;
R(10) is hydrogen or alkyl having 1 or 2 carbon atoms;
R(11) is cycloalkyl having 3, 4, 5 or 6 carbon atoms, phenyl, where phenyl is unsubstituted or substituted by 1, 2 or 3 substituents selected from the group consisting of F, Cl, CF₃, OCF₃, CN, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is CₚH₂ₚ-R(14');
p is 0, 1, 2, 3 or 4;
R(14') is aryl or heteroaryl,
where aryl and heteroaryl are unsubstituted or substituted by 1, 2 or 3 substituents selected from the group consisting of F, Cl, CF₃, OCF₃, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen;
R(3) is alkyl having 3, 4 or 5 carbon atoms, phenyl, where phenyl is unsubstituted or substituted by 1, 2 or 3 substituents selected from the group consisting of F, Cl, CF₃, OCF₃, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

7. Compound of the formula I according to one or more of Claims 1 to 3 and 5, which is

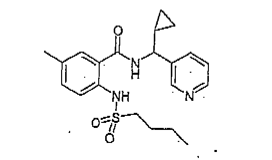

8. Compound of the formula I according to one or more of Claims 1 to 3 and 5, which is

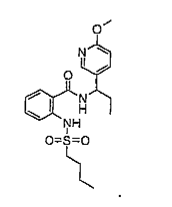

9. Compounds of the formula I according to one or more of Claims 1 to 8 and the physiologically tolerated salts thereof for use as pharmaceuticals.

10. Pharmaceutical preparation comprising an effective amount of at least one compound of the formula I according to one or more of Claims 1 to 8 and/or of a physiologically tolerated salt thereof as active ingredient together with pharmaceutically acceptable carriers and additives and, where appropriate, also one or more other pharmacological active ingredients.

11. Use of a compound of the formula I according to one or more of Claims 1 to 8 and/or of a physiologically tolerated salt thereof for producing a medicament with K⁺ channel-blocking effect for the therapy and prophylaxis of K⁺ channel-mediated diseases.

12. Use of a compound of the formula I according to one or more of Claims 1 to 8 and/or of a physiologically tolerated salt thereof for producing a medicament for the therapy or prophylaxis of cardiac arrhythmias which can be abolished by prolonging the action potential.

13. Use of a compound of the formula I according to one or more of Claims 1 to 8 and/or of a physiologically tolerated salt thereof for producing a medicament for the therapy or prophylaxis of reentry arrhythmias.

14. Use of a compound of the formula I according to one or more of Claims 1 to 8 and/or of a physiologically tolerated salt thereof for producing a medicament for the therapy or prophylaxis of supraventricular arrhythmias.

15. Use of a compound of the formula I according to one or more of Claims 1 to 8 and/or of a physiologically tolerated salt thereof for producing a medicament for the therapy or prophylaxis of atrial fibrillation or atrial flutter.

16. Pharmaceutical composition comprising an effective amount of at least one compound of the formula I according to one or more of Claims 1 to 8 and/or of a physiologically tolerated salt thereof and of a betablocker as active ingredients together with pharmaceutically acceptable carriers and additives.

17. Pharmaceutical composition comprising an effective amount of at least one compound of the formula I according to one or more of Claims 1 to 8 and/or of a physiologically tolerated salt thereof and of an IKs channel blocker as active ingredients together with pharmaceutically acceptable carriers and additives.

## Revendications

1. Composés de formule I, 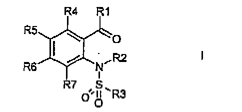 où
R1 signifie 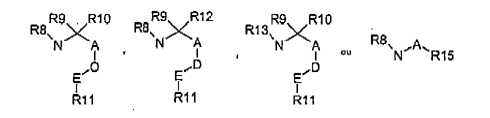
A signifie ;
n vaut 0, 1, 2, 3, 4 ou 5 ;
O signifie oxygène ;
D signifie une liaison ou oxygène ;
E signifie -CₘH₂ₘ-;
m vaut 0, 1, 2, 3, 4 ou 5 ;
R(8) signifie hydrogène, alkyle comprenant 1, 2, 3 ou 4 atomes de carbone ou CₚH₂ₚ-R(14) ;
p vaut 0, 1, 2, 3, 4 ou 5 ;
R(14) signifie cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(9) signifie hydrogène ou alkyle comprenant 1, 2, 3, 4, 5 ou 6 atomes de carbone ;
R(10) signifie hydrogène, alkyle comprenant 1, 2, 3 ou 4 atomes de carbone, cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, T, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbon, diméthylamino, sulfamoyle, méthylsulfonyle et méthylsulfonylamino ;
R(11) signifie cycloalkyle comprenant 3, 4, 5 ou 6 atomes de carbone, phényle, naphtyle, thiényle, furyle, pyridyle, pyrazinyle, pyridazinyle ou pyrimidyle, où phényle, naphtyle, thiényle, furyle, pyridyle, pyrazinyle, pyridazinyle et pyrimidyle sont non substitués ou substitués par 1, 2 ou 3 substituants choisis dans le groupe constitué par F, Cl, Br, I, CF₃, OCF, NO₂, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
signifie alkyle comprenant 1, 2, 3 ou 4 atomes de carbone, cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚH₂ₚ-R(14') ;
p vaut 0, 1, 2, 3, 4 ou 5 ;
R(14') signifie cycloalkyle comprenant 3, 4, 5 ou 6 atomes de carbone, tétrahydrofurannyle, tétrahydropyrannyle, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(15) signifie cycloalkyle comprenant 3, 4, 5, 6, 7 ou 8 atomes de carbone ;
R(2) signifie hydrogène ou alkyle comprenant 1, 2, 3 ou 4 atomes de carbone ;
R(3) signifie alkyle comprenant 3, 4, 5, 6 ou 7 atomes de carbone, cycloalkyle comprenant 3, 4, 5 ou 6 atomes de carbone, phényle ou naphtyle, où phényle ou naphtyle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un un de l'autre hydrogène, F, Cl, Br, I, CF₃, OCF₃, OCHF₂, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
où les radicaux cycloalkyle peuvent être ramifiés ;
ainsi que leurs sels pharmaceutiquement acceptables.

2. Composés de formule I selon la revendication 1, dans laquelle :
R(1) signifie 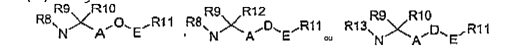
A signifie -CₙH₂ₙ- ;
n vaut 0, 1, 2, 3, 4 ou 5 ;
O signifie oxygène ;
D signifie une liaison ou oxygène :
E signifie -CₘH₂ₘ-;
m vaut 0, 1, 2, 3, 4 ou 5 ;
R(8) signifie hydrogène, alkyle comprenant 1, 2, 3 ou 4 atomes de carbone ou CₚH₂ₚ-R(14) ;
p vaut 0, 1, 2, 3, 4 ou 5;
R(14) signifie cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(9) signifie hydrogène ou alkyle comprenant 1, 2, 3, 4, 5 ou 6 atomes de carbone ;
R(10) signifie hydrogène, alkyle comprenant 1, 2, 3 ou 4 atomes de carbone, cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(11) signifie cycloalkyle comprenant 3, 4, 5 ou 6 atomes de carbone, phényle ou pyridyle, où phényle et pyridyle sont non substitués ou substitués par 1, 2 ou 3 substituants choisis dans le groupe constitué par F, Cl, Br, I, CF₃, OCF₃, NO₂, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(12) signifie alkyle comprenant 1, 2, 3 ou 4 atomes de carbone, cycloalkyle comprenant 3, 4, 5 ou 6 atomes de carbone, aryle ou hétéroaryle, où aryle et hétéroaryle sont non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚX₂ₚ-R(14') ;
p vaut 0, 1, 2, 3, 4 ou 5 ;
R(14') signifie cycloalkyle comprenant 3, 4, 5 ou 6 atomes de carbone, tétrahydrofurannyle, tétrahydropyrannyle, aryle ou hétéroaryle, où aryle et hétéroaryle sont non substitués ou substitués par 1, 2 ou 3 substituant choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1, 2, 3 ou 4 atomes de carbone ;
R(3) signifie alkyle comprenant 3, 4 ou 5 atomes de carbone, phényle, où phényle est non substitué ou substitué par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, Cl, Br, I, CF₃, OCF₃, OCHF₂, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
ainsi que leurs sels pharmaceutiquement acceptables.

3. Composés de formule I selon la revendication 1 ou 2, dans laquelle :
R(1) signifie 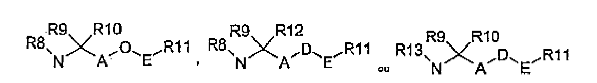
A signifie -CₙH₂ₙ- ;
n vaut 0, 1, 2 ou 3 ;
O signifie oxygène ;
D signifie une liaison ou oxygène ;
E signifie -CₘH₂ₘ-;
m vaut 0, 1, 2 ou 3 ;
R(8) signifie hydrogène ou alkyle comprenant 1, 2, 3 ou 4 atomes de carbone ;
R(9) signifie hydrogène ou alkyle comprenant 1, 2, 3, 4, 5 ou 6 atomes de carbone ;
R(10) signifie hydrogène, alkyle comprenant 1, 2, 3 ou 4 atomes de carbone ou cycloalkyle comprenant 3, 4 ou 5 atomes de carbone ;
R(11) signifie cycloalkyle comprenant 3, 4, 5 ou 6 atomes de carbone, phényle ou pyridyle, où phényle et pyridyle sont non substitués ou substitués par 1, 2 ou 3 substituants choisis dans le groupe constitué par F, Cl, Br, CF₃, OCF₃, NO₂, CN, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(12) signifie alkyle comprenant 1, 2, 3 ou 4 atomes de carbone, cycloalkyle comprenant 3, 4 ou 5 atomes de carbone, aryle ou hétéroaryle, où aryle et hétéroaryle sont non substitués ou substitués par 1, 2 ou 3 substituants choisis dans le groupe constitué par F, Cl, Br, CF₃, OCF₃, NO₂, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚH₂ₚ-R(14') ;
p vaut 0, 1, 2, 3, 4 ou 5 ; R(14') signifie cycloalkyle comprenant 3, 4, 5 ou 6 atomes de carbone, tétrahydrofurannyle, tétrahydropyrannyle, aryle ou hétéroaryle, où aryle et hétéroaryle sont non substitués ou substitués par 1, 2 ou 3 substituant choisis dans le groupe constitué par F, Cl, Br, CF₃, OCF₃, NO₂, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1 ou 2 atomes de carbone ;
R(3) signifie alkyle comprenant 3, 4 ou 5 atomes de carbone, phényle,
où phényle est non substitué ou substitué par 1, 2 ou 3 substituants choisis dans le groupe constitué par F, Cl, Br, CF₃, OCF₃, NO₂, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, Cl, Br, CF₃, OCF₃, OCHF₂, NO₂, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthvlsulfonylamino ;
ainsi que leurs sels pharmaceutiquement acceptables.

4. Composés de formule I selon les revendications 1-3, dans laquelle :
R(1) signifie 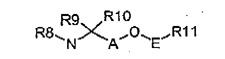
A signifie -CₙH₂ₙ- ;
n vaut 0, 1 ou 2 ;
O signifie oxygène ;
E signifie -CₘH₂ₘ-;
m vaut 0 ou 1 ;
R(8) signifie hydrogène ou alkyle comprenant 1, 2, 3 ou 4 atomes de carbone ;
R(9) signifie hydrogène ou alkyle comprenant 1, 2, 3 ou 4 atomes de carbone ;
R(10) signifie hydrogène ou alkyle comprenant 1 ou 2 atomes de carbone ;
R(11) signifie phényle, où phényle est non substitué ou substitué par 1, 2 ou 3 substituants choisis dans le groupe constitué par F, Cl, CF₃, OCF₃, CK, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ;
R(3) signifie alkyle comprenant 3, 4 ou 5 atomes de carbone, phényle, où phényle est non substitué ou substitué par 1, 2 ou 3 substituants choisis dans le groupe constitué par F, Cl, CF₃, OCF₃, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, Cl, CF₃, OCF₃, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
ainsi que leurs sels pharmaceutiquement acceptables.

5. Composés de formule I selon les revendications 1-3, dans laquelle :
R1 signifie 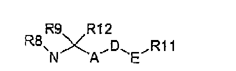
A signifie -CₙH₂ₙ-;
n vaut 0, 1 ou 2 ;
D signifie une liaison ou oxygène ;
E signifie -CₘH₂ₘ- ;
m vaut 0 ou 1 ;
R(B) signifie hydrogène ou allyle comprenant 1, 2, 3 ou 4 atomes de carbone ;
R(9) signifie hydrogène ou alkyle comprenant 1, 2, 3 ou 4 atomes de carbone ;
R(11) signifie phényle ou pyridyle, où phényle et pyridyle sont non substitués ou substitués par 1, 2 ou 3 substituants choisis dans le groupe constitué par F, Cl , CF₃, OCF₃, CN, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(12) signifie alkyle comprenant 1, 2, 3 ou 4 atomes de carbone ou cyclopropyle ;
R(2) signifie hydrogène ;
R(3) signifie alkyle comprenant 3, 4 ou 5 atomes de carbone, phényle, où phényle est non substitué ou substitué par 1, 2 ou 3 substituants choisis dans le groupe constitué par F, Cl, CF₃, OCF₃, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, Cl, CF₃, OCF₃, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
ainsi que leurs sels pharmaceutiquement acceptables.

6. Composés de formule I selon les revendications 1-3, dans laquelle :
R1 signifie 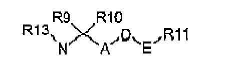 signifie -CₙH₂ₙ- ;
n vaut 0, 1 ou 2 ;
D signifie une liaison ou oxygène ;
E signifie -CₘH₂ₘ-;
m vaut 0 ou 1;
R(9) signifie hydrogène ou alkyle comprenant 1 ou 2 atomes de carbone ;
R(10) signifie hydrogène ou alkyle comprenant 1 ou 2 atomes de carbone ;
R(11) signifie cycloalkyle comprenant 3, 4, 5 ou 6 atomes de carbone, phényle, où phényle est non substitué ou substitué par 1, 2 ou 3 substituants choisis dans le groupe constitué par F, Cl, CF₃, OCX₃, CN, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚH₂ₚ-R(14') ;
p vaut 0, 1, 2, 3 ou 4;
R(14') signifie aryle ou hétéroaryle, où aryle et hétéroaryle sont non substitués ou substitués par 1, 2 ou 3 substituants choisis dans le groupe constitué par F, Cl, CF₃, OCF₃, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ;
R(3) signifie alkyle comprenant 3, 4 ou 5 atomes de carbone, phényle, où phényle est non substitué ou substitué par 1, 2 ou 3 substituant choisis dans le groupe constitué par F, Cl, CF₃, OCF₃, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, Cl, CF₃, OCF₃, CN, COOMe, CONH₂, COMe, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
ainsi que leurs sels pharmaceutiquement acceptables.

7. Composés de formule I selon l'une ou plusieurs des revendications 1 à 3 et 5, qui est

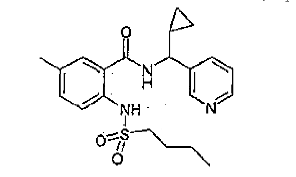

8. Composés de formule I selon l'une ou plusieurs des revendications 1 à 3 et 5, qui est

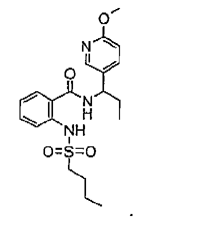

9. Composés de formule I selon l'une ou plusieurs des revendications 1 à 8 et leurs sels physiologiquement acceptables pour une utilisation comme médicament.

10. Préparation pharmaceutique, contenant une quantité active d'au moins un composé de formule I selon l'une ou plusieurs des revendications 1 à 8 et/ou d'un sel physiologiquement acceptable de celui-ci comme substance active avec des supports et des additifs pharmaceutiquement acceptables et le cas échéant encore une ou plusieurs autres substances actives pharmacologiques.

11. Utilisation d'un composé de formule I selon l'une ou plusieurs des revendications 1 à 8 et/ou d'un sel physiologiquement acceptable de celui-ci pour la préparation d'un médicament présentant un effet de blocage du canal K⁺ destiné à la thérapie et la prophylaxie de maladies dans lesquelles intervient le canal K⁺.

12. Utilisation d'un composé de formule I selon l'une ou plusieurs des revendications 1 à 8 et/ou d'un sel physiologiquement acceptable de celux-ci pour la préparation d'un médicament destiné à la thérapie ou la prophylaxie de troubles du rythme cardiaque, qui peuvent être supprimés par un allongement du potentiel d'action.

13. Utilisation d'un composé de formule I selon l'une ou plusieurs des revendications 1 à 8 et/ou d'un sel physiologiquement acceptable de celui-ci pour la préparation d'un médicament destiné à la thérapie ou la prophylaxie d'arythmies par réentrée.

14. Utilisation d'un composé de formule I selon l'une ou plusieurs des revendications 1 à 8 et/ou d'un sel physiologiquement acceptable de celui-ci pour la préparation d'un médicament destiné à la thérapie ou la prophylaxie d'arythmies supraventriculaires.

15. Utilisation d'un composé de formule I selon l'une ou plusieurs des revendications 1 à 8 et/ou d'un sel physiologiquement acceptable de celui-ci pour la préparation d'un médicament destiné à la thérapie ou la prophylaxie d'une fibrillation atriale ou d'une arythmie atriale.

16. Préparation pharmaceutique, contenant une quantité active d'au moins un composé de formule I selon l'une ou plusieurs des revendications 1 à 8 et/ou d'un sel physiologiquement acceptable de celui-ci ainsi qu'un bêtabloquant comme substances actives, avec des supports et des additifs pharmaceutiquement acceptables.

17. Préparation pharmaceutique, contenant une quantité active d'au moins un composé de formule I selon l'une ou plusieurs des revendications 1 à 8 et/ou d'un sel physiologiquement acceptable de celui-ci ainsi qu'un bloquant du canal IKs comme substances actives, avec des supports et des additifs pharmaceutiquement acceptables.
